# EUROPÄISCHE PATENTANMELDUNG

(11) **EP 4 635 395 A1**
(43) Veröffentlichungstag der Anmeldung: **22.10.2025**
(21) Anmeldenummer: 25170818.6
(22) Anmeldetag: 15.04.2025
(51) Int. Cl.: A61B 1/00, A61B 1/04, G02B 7/00, G02B 26/00

(54) **FILTERWECHSELVORRICHTUNG FÜR EINE ENDOSKOPISCHE KAMERA, KAMERAKOPF FÜR EIN ENDOSKOP UND NACHRÜSTSATZ ZUM NACHRÜSTEN EINES KAMERAKOPFES UND/ODER EINES ENDOSKOPS**

(30) Priorität: 18.04.2024 DE 102024110880
(71) Anmelder: Karl Storz SE & Co. KG, 78532 Tuttlingen (DE)
(72) Erfinder: DAVID, Jonas, 78532 Tuttlingen (DE)

(57) **Zusammenfassung**

Die Erfindung betrifft eine Filterwechselvorrichtung für eine endoskopische Kamera, wobei ein erstes Drehplattenlager mit zwei Aussparungen zum Durchführen von jeweils einem Führungselement zwischen einem ersten Gehäuseteil und einer ersten Plattenfläche einer Drehplatte angeordnet ist, die Drehplatte relativ zum mindestens ersten Gehäuseteil drehbar ist, mindestens zwei schwenkbaren Filterhalter jeweils mittels einer Rotationsachse drehbar an dem ersten Gehäuseteil angeordnet sind und jeweils ein Führungselement ausgerichtet zur Drehplatte aufweisen, wobei die Drehplatte auf ihrer ersten Plattenfläche mindestens eine erste Führungsebene mit einer geformten, umlaufenden Kontaktfläche an einem Umfang aufweist, sodass beim Drehen der Drehplatte durch eine Druckkraft der geformten, umlaufenden Kontaktfläche auf zumindest einem der beiden Führungselemente der zugehörige Filterhalter in oder aus den optischen Durchgang schwenkbar oder in einer Ausgangsposition frei von dem optischen Durchgang positionierbar ist. Des Weiteren betrifft die Erfindung einen Kamerakopf und einen Nachrüstsatz zum Nachrüsten eines Kamerakopfes und/oder eines Endoskopes.

## Beschreibung

Die Erfindung betrifft eine Filterwechselvorrichtung für eine endoskopische Kamera, wobei die Filterwechselvorrichtung mindestens ein erstes Gehäuseteil, eine Drehplatte mit zwei sich gegenüberliegenden Plattenflächen, mindestens ein erstes Drehplattenlager und mindestens zwei schwenkbare Filterhalter mit jeweils einer Filteraufnahme für einen optischen Filter aufweist, wobei das erste Drehplattenlager zwischen dem ersten Gehäuseteil und einer ersten Plattenfläche der Drehplatte angeordnet ist, die Drehplatte relativ zum mindestens ersten Gehäuseteil drehbar ist und das mindestens erste Gehäuseteil, das mindestens erste Drehplattenlager und die Drehplatte jeweils einen optischen Durchgang entlang einer optischen Achse aufweisen, wobei die mindestens zwei schwenkbaren Filterhalter jeweils mittels einer Rotationsachse drehbar an dem ersten Gehäuseteil angeordnet sind und jeweils ein Führungselement ausgerichtet zur Drehplatte aufweisen. Des Weiteren betrifft die Erfindung einen Kamerakopf für ein Endoskop und einen Nachrüstsatz zum Nachrüsten eines Kamerakopfes und/oder eines Endoskopes.

In medizinischen und nicht-medizinischen Anwendungen werden Beobachtungsinstrumente wie Endoskope eingesetzt, um innere Hohlräume eines menschlichen oder tierischen Körpers oder eines industriellen, technischen Objektes, wie eine Rohrleitung, zu untersuchen. Für die Bildgebung kann ein Kamerakopf aufweisend einen Bildsensor zusammen mit dem Endoskop eingesetzt werden. Um die Bildqualität zu verbessern und/oder verschiedene Beobachtungsmodi zu ermöglichen, ist es bekannt, verschiedene Filter in den Strahlengang des Beobachtungsinstrumentes einzubringen.

Zum Beispiel wird in der Fluoreszenz-Bildgebung das zu untersuchende Objekt einem Licht mit einer Anregungsstrahlung ausgesetzt, welche ein vorher auf das Objekt aufgebrachtes oder bereits vorhandenes Fluorophor anregt, Licht einer bestimmten Emissionswellenlänge auszusenden, wobei die Anregungswellenlänge und die Emissionswellenlänge sich üblicherweise unterscheiden. Normalerweise ist die Emissionswellenlänge länger als die Anregungswellenlänge. Das abgegebene Emissionslicht ist üblicherweise deutlich schwächer als andere Lichtquellen, wie das Anregungsfluoreszenzlicht oder das bildgebende Weißlicht. Aus diesen Gründen ist es notwendig, unerwünschte Wellenlängenbänder mittels eines Filters herauszufiltern, sodass möglichst nur das gewünschte Spektrum und/oder die Emissionswellenlänge des Fluorophors den Kamerakopf im Fluoreszenzmodus erreicht. Zum Wechsel zwischen verschiedenen Beobachtungsmodi werden üblicherweise zwei oder mehrere Filter nacheinander in den Strahlengang der Beobachtungsoptik eingebracht, wozu prinzipiell verschiedene Filterwechsler bekannt sind.

DE 101 57 057 A1 offenbart eine Vorrichtung zum Positionieren zumindest eines optischen Bauelementes innerhalb eines endoskopischen Systems mit einem Gehäuse, durch welches die optische Achse des endoskopischen Systems verläuft und in dem das zumindest eine Bauelement angeordnet ist, welches um eine im Wesentlichen parallel zu einer Längsachse des Gehäuses verlaufenden Schwenkachse in den Strahlengang einschwenkbar und aus diesem wieder ausschwenkbar ist, wobei das zumindest eine Bauelement, beispielsweise ein Filter für einen speziellen spektralen Wellenlängenbereich, an einem um die Schwenkachse schwenkbaren Träger angeordnet ist. Hierbei ist ein kleinster Abstand einer Innenwand des Gehäuses von der Schwenkachse kleiner als ein größter Abstand der Schwenkachse zu einer Außenkante des zumindest einen Bauelementes. Durch die nähere Anordnung der Schwenkachse jedes schwenkbaren Trägers zu der Innenwand des Gehäuses ist die Anzahl der Träger zum Halten möglichst unterschiedlicher Filter durch die räumlich weitreichende Schwenkbewegung innerhalb des limitierten Raumes des Gehäuses begrenzt. Zudem ist das Gehäuse fest mit einem Gehäuse des optischen Kopfes des Endoskopes verbunden. Weiterhin nachteilig ist, dass durch räumlich nähere Anordnung der Schwenkachse an der Innenwand des Gehäuses eine Vielzahl von beweglichen Einzelteilen erforderlich ist, um mehrere Filter in den Strahlengang einzubringen. Dies hat einen erhöhten Kosten- und Montageaufwand zur Folge. Zudem erhöht sich dadurch das Risiko von Verschleiß, Ungenauigkeiten in der jeweiligen Schwenkbewegung und folglich von Fehlfunktionen.

Aus dem anmeldereigenen Stand der Technik (deutsches Anmeldeaktenzeichen 10 2022 131 502.9) ist eine Filterwechselvorrichtung für einen endoskopischen Kamerakopf mit mindestens drei optischen Filtern bekannt, welche eine Nut mit einer nicht kreisförmigen Führungsbahn und einem Drehelement aufweist, wobei durch Drehen des Drehelementes nacheinander in der Nut angeordnete Träger für jeweils ein Filter bewegt oder eine Schwenkbewegung eines Trägerarms mittels eines partiell in der Nut angeordneten Führungselementes aufgrund einer zumindest partiellen Bewegung entlang der nicht kreisförmigen Führungsbahn in und aus dem Strahlengang erfolgt. Nachteilig hierbei ist, dass zur Anordnung von mehreren Filtern jeweils ein Trägerarm mit einem Führungselement notwendig ist, sodass es aufgrund der größeren Anzahl von Führungselementen oder direkt in der Nut angeordneten Filterträgern aufgrund der auftretenden Reibungskräfte zu störenden Einflüssen auf die Funktion der Filterwechselvorrichtung kommen kann. Hierbei können die hohen Reibungskräfte ein Verklemmen des Bewegungsmechanismus und/oder einen Verschleiß mit negativer Beeinflussung auf das Ein- und Ausschwenkverhalten und eine Rastposition bewirken, sodass eine Fehlfunktion oder ein Defekt der Filterwechselvorrichtung auftreten kann. Zudem überlappen sich die schwenkbaren Trägerarme, sodass entlang der optischen Achse ein größerer Bauraum notwendig ist.

Aufgabe der Erfindung ist es, den Stand der Technik zu verbessern.

Gelöst wird die Aufgabe durch eine Filterwechselvorrichtung für eine endoskopische Kamera, wobei die Filterwechselvorrichtung mindestens ein erstes Gehäuseteil, eine Drehplatte mit zwei sich gegenüberliegenden Plattenflächen, mindestens ein erstes Drehplattenlager und mindestens zwei schwenkbare Filterhalter mit jeweils einer Filteraufnahme für einen optischen Filter aufweist, wobei das erste Drehplattenlager zwischen dem ersten Gehäuseteil und einer ersten Plattenfläche der Drehplatte angeordnet ist, die Drehplatte relativ zum mindestens ersten Gehäuseteil drehbar ist und das mindestens erste Gehäuseteil, das mindestens erste Drehplattenlager und die Drehplatte jeweils einen optischen Durchgang entlang einer optischen Achse aufweisen, wobei die mindestens zwei schwenkbaren Filterhalter jeweils mittels einer Rotationsachse drehbar an dem ersten Gehäuseteil angeordnet sind und jeweils ein Führungselement ausgerichtet zur Drehplatte aufweisen, und das mindestens erste Drehplattenlager zwei Aussparungen zum Durchführen von jeweils einem Führungselement aufweist und die Drehplatte auf ihrer ersten Plattenfläche mindestens eine erste Führungsebene mit einer geformten, umlaufenden Kontaktfläche an einem Umfang aufweist, sodass beim Drehen der Drehplatte durch eine Druckkraft der geformten, umlaufenden Kontaktfläche auf zumindest einem der beiden Führungselemente der zugehörige Filterhalter in oder aus dem optischen Durchgang schwenkbar oder in einer Ausgangsposition frei von dem optischen Durchgang positionierbar ist.

Somit wird eine Filterwechselvorrichtung bereitgestellt, mit welcher mindestens zwei oder mehrere Filter einzeln nacheinander in den Strahlengang einer endoskopischen Kamera schnell, präzise und effizient ein- und ausschwenkbar sind. Folglich ist ein schneller und dennoch eindeutiger Wechsel zwischen verschiedenen Filtern ermöglicht. Vor allem sind durch das einfache Drehen im Uhrzeigersinn und/oder gegen den Uhrzeigersinn eine vorgegebene Abfolge der Filter und ein schneller Wechsel zwischen verschiedenen Filtern und somit schnell nacheinander verschiedene Beobachtungsmodi und Bildgebungen ermöglicht.

Es ist besonders vorteilhaft, dass beim Drehen der Drehplatte und Einwirken der Druckkraft der geformten, umlaufenden Kontaktfläche auf zumindest ein Führungselement eine geringe Reibungskraft auftritt, da die Druckkraft nur partiell an der Außenoberfläche des Führungselementes und insbesondere jeweils abschnittsweise und/oder einseitig auf die Außenoberfläche des Führungselementes wirkt. Aufgrund der kleinflächigen Druckkraftübertragung wird ein Verschleiß und folglich eine Fehlfunktion oder ein Defekt der Filterwechselvorrichtung verhindert. Dadurch, dass das jeweilige Führungselement sich bei der Rotation der Drehplatte entlang der geformten, umlaufenden Kontaktfläche der jeweiligen Führungsebene bewegt, findet zudem ein kontinuierlicher Wechsel des Oberflächenbereiches statt, auf welchem jeweils die Druckkraft der geformten, umlaufenden Kontaktfläche einwirkt. Folglich findet entsprechend der Ein- und Ausschwenkbewegung ein kontinuierlicher Wechsel der druckbelasteten Oberfläche des jeweiligen Führungselementes statt. Dadurch wird eine präzise Bewegung des jeweiligen Führungselementes entlang der geformten, umlaufenden Kontaktfläche der Führungsebene und folglich ein sicheres und präzises Ein- und Ausschwenken der Filteraufnahme und/oder eines optischen Filters des jeweiligen schwenkbaren Filterhalters in den optischen Durchgang gewährleistet.

Dadurch, dass lediglich die Materialdicken der Drehplatte und des jeweiligen Drehplattenlagers entlang der optischen Achse angeordnet sind, weist die Filterwechselvorrichtung eine geringe axiale Ausdehnung und somit eine geringe Baugröße entlang der optischen Achse auf.

Weiterhin ist besonders vorteilhaft, dass durch Ausbildung der Drehplatte mit mindestens einer ersten Führungsebene mit einer geformten, umlaufenden Kontaktfläche, ein Ein- und Ausschwenken der Filterhalter sowohl beim Drehen im Uhrzeigersinn als auch gegen den Uhrzeigersinn ermöglicht und die Drehrichtung jederzeit wechselbar ist. Dadurch, dass die geformte Kontaktfläche der jeweiligen Führungsebene umlaufend und somit ohne ein Ende ausgebildet ist, ist die Drehplatte beständig von einem Anwender durchrotierbar und das jeweilige Führungselement ist unbegrenzt mittels der Druckkraft der geformten, umlaufenden Kontaktfläche räumlich und/oder zweidimensional bewegbar, wodurch dem zugehörigen Filterhalter eine entsprechende Einschwenk- oder Ausschwenkbewegung oder eine Ausgangs- oder Übergangsposition aufgeprägt wird. Somit führt der jeweilige, zugehörige Filterhalter eine bestimmte vorgegebene Bewegung aus, verbleibt in einer Ausgangsposition oder nimmt eine Ausgangsposition ein, sodass die Position und Bewegung der mindestens zwei schwenkbaren Filterhalter optimal zeitlich und räumlich aufeinander abstimmbar sind. Dadurch ist es ermöglicht, dass mindestens zwei Filterhalter in einer Ebene und/oder nebeneinander an einer Plattenfläche der Drehplatte angeordnet sind.

Durch die kompakte Bauform der Drehplatte und des mindestens einen Drehplattenlagers und durch die aufeinander abgestimmten räumlichen Anordnungen und Bewegungen der Filterhalter ist eine sehr kompakte Bauform der Filterwechselvorrichtung realisiert. Vor allem weist die Filterwechselvorrichtung aufgrund der Anordnung der mindestens zwei Filterhalter in einer Ebene eine geringe Abmessung entlang der optischen Achse auf.

Es ist besonders vorteilhaft, dass durch die Form, Geometrie und den Verlauf der jeweiligen Führungsebene mit der geformten, umlaufenden Kontaktfläche gezielt dem jeweiligen Führungselement und somit dem zugehörigen Filterhalter, welcher um seine Rotationsachse dreh- und schwenkbar ist, definiert eine Einschwenk-, Ausschwenk-, Übergangs-, Ausgangs- und/oder Ruheposition und/oder -bewegung vorgegeben ist. Durch die Geometrie und den umlaufenden Verlauf der speziell geformten, umlaufenden Kontaktfläche der jeweiligen Führungsebene erfolgt eine Festlegung einer definierten Ausrichtung und/oder Position des jeweiligen Filterhalters zur optischen Achse und/oder dem optischen Durchgang und wird eine gleichzeitige Bewegung von mindestens zwei Filterhaltern auf einer der beiden Plattenflächen der Drehplatte ermöglicht. Somit werden durch den Verlauf der geformten, umlaufenden Kontaktfläche der jeweiligen Führungsebene definierte, aufeinanderfolgende Bewegungen für das jeweilige Führungselement in Kontakt mit dieser geformten, umlaufenden Kontaktfläche vorgegeben. Durch die Ausgestaltung und Form der geformten, umlaufenden Kontaktfläche wird ein nacheinander folgendes, einzelnes Einschwenken der Filterhalter ermöglicht, ohne dass die Gefahr besteht, dass sich die bewegenden Filterhalter auf einer Platenfläche der Drehplatte berühren. Folglich wird die räumliche Ausrichtung von mindestens zwei an einer Plattenfläche angeordneten Filterhalter durch die Ausgestaltung der geformten, umlaufenden Kontaktfläche der jeweiligen Führungsebene und dadurch der zeitliche Ablauf der Bewegung des jeweiligen Filterhalters und der Wechsel der Filter vorgegeben.

Um die aufgeprägten Druckkraft durch die geformte, umlaufende Kontaktfläche der jeweiligen Führungsebene auf das jeweilige Führungselement gezielt in eine gewünschte Schwenkbewegung oder Einhaltung einer Ausgangsposition umzusetzen, sind die mindestens zwei Filterhalter mittels ihrer jeweiligen Rotationsachsen in einem räumlich vorgegebenen Abstand und in einer räumlichen Position zueinander und/oder zu der geformten, umlaufenden Kontaktfläche angeordnet.

Ein wesentlicher Gedanke der Erfindung beruht darauf, mindestens zwei um ihre jeweilige Rotationsachse schwenkbare Filterhalter an einem distalseitigen oder proximalseitigen Gehäuseteil anzuordnen, wobei ein jeweiliges Führungselement des jeweiligen Filterhalters nach innen gerichtet ist und durch eine zugehörige Aussparung eines Drehplattenlagers durchtritt, welches zwischen dem Gehäuseteil und einer Drehplatte angeordnet ist, wobei die Drehplatte auf ihrer den Führungselementen zugewandten Plattenfläche mindestens eine Führungsebene mit einer geformten, umlaufenden Kontaktfläche aufweist, sodass beim Drehen der Drehplatte die geformte, umlaufende Kontaktfläche eine Druckkraft auf das jeweilige Führungselement auswirkt, wodurch das Führungselement räumlich bewegt und dem zugehörigen Filterhalter eine Schwenkbewegung um seine Rotationsachse oder eine Ausgangsposition aufprägbar ist. Durch die Form und den Verlauf der geformten, umlaufenden Kontaktfläche werden mittels der Druckkraft und dem zugehörigen Führungselement den jeweiligen Filterhaltern eine räumliche Ausrichtung und/oder Bewegung relativ zu dem optischen Durchgang vorgegeben. In Zusammenwirken mit der schwenkbaren Anordnung der mindestens zwei Filterhalter an dem Gehäuseteil mittels einer jeweiligen Rotationsachse werden ein zeitlicher Verlauf und eine Reihenfolge des Wechsels der Filter beim Drehen der Drehplatte aufgeprägt. Somit wird eine Filterwechselvorrichtung bereitgestellt, mit welcher verschiedene Filter, insbesondere mehrere Fluoreszenzfilter und/oder ein Weißlichtfilter, in den Strahlengang einer endoskopischen Kamera präzise, schnell, effizient und mit einer langen Standzeit der Filterwechselvorrichtung ein- und ausschwenkbar sind.

### Folgendes Begriffliche sei erläutert:

Eine "Filterwechselvorrichtung" ist insbesondere eine Vorrichtung, mit welcher zumindest ein Filter von zwei oder mehreren Filtern in und aus dem optischen Strahlengang bewegbar ist. Mittels der Filterwechselvorrichtung werden zwei oder mehrere Filter insbesondere einzeln und nacheinander in den optischen Durchgang eingeschwenkt und/oder wieder ausgeschwenkt. Die Filterwechselvorrichtung ist insbesondere manuell oder automatisch aktivierbar zum Wechseln der Filter durch Drehen der drehbaren Drehplatte. Somit ist jeweils ein Filter automatisch oder manuell durch Drehen in den optischen Durchgang ein- und ausschwenkbar. Hierdurch kann ein Wechsel zwischen mindestens zwei Filteraufnahmen und/oder Filtern zweier Filterhalter erfolgen, deren Führungselemente an der Kontaktfläche derselben Führungsebene, zweier unterschiedlicher Führungsebenen einer Plattenfläche oder einer Führungsebene der ersten Plattenfläche und einer Führungsebene der zweiten Plattenfläche angeordnet sind und/oder mittels der Druckkraft der jeweiligen Führungsebene beaufschlagt werden. Die Filterwechselvorrichtung weist insbesondere mindestens zwei optische Filter, bevorzugt mindestens drei oder vier und optional weitere optische Filter, auf. Optional kann die Filterwechselvorrichtung auch eine Filteraufnahme aufweisen, welche kein optisches Filter aufweist und somit einen freien Durchlass durch den optischen Strahlengang ermöglicht. Somit kann mittels der Filterwechselvorrichtung auch eine leere Filteraufnahme in den optischen Durchgang und in den Strahlengang eingebracht werden. Ebenso kann der freie Durchgang statt Weglassen eines optischen Filters auch durch ein nicht filtrierendes optisches Element, wie eine Glasscheibe, ermöglicht sein. Eine Glasscheibe als Fenster kann auch eine Anti-Reflexions-Beschichtung aufweisen. Die Filterwechselvorrichtung kann insbesondere in einer Kamera integriert sein oder als separate Vorrichtung, beispielsweise ausgebildet als Aufschnappfilter, mit der Kamera und/oder einem Endoskop verbindbar sein. Für einen automatischen Filterwechsel kann die Filterwechselvorrichtung ein Bedienelement, beispielsweise einen Schalter an ihrer Außenoberfläche, aufweisen. Alternativ oder ergänzend zu einer optischen Erkennung des Filterwechsels durch den Anwender kann die Filterwechselvorrichtung auch ein Anzeigeelement und/oder einen Sensor, beispielsweise einen Hall-Sensor, aufweisen.

Ein "optisches Filter" (vereinfacht auch als "Filter" bezeichnet) ist insbesondere ein optisches Element, welches die einfallende Strahlung und/oder einfallenden Strahlen basierend auf spezifischen Eigenschaften, wie beispielsweise einer Wellenlänge, einem Polarisationszustand, einem Einfallswinkel und/oder einer Einfallsrichtung, selektiert und somit durchlässt oder am Durchlassen hindert. Ebenso kann ein optisches Filter die Eigenschaften des durchtretenden Lichtes verändern, beispielsweise durch Umwandlung von kreisförmig polarisiertem Licht in linearpolarisiertem Licht. Insbesondere kann ein optisches Filter ein spezifisches spektrales Wellenlängenband blockieren. Ein optisches Filter kann beispielsweise ein Verlaufsfilter, ein Kantenfilter, ein Polarisationsfilter oder ein Interferenzfilter sein. Ein Interferenzfilter weist insbesondere eine Beschichtung auf, welche ein Blockieren oder Durchlassen von Licht eines bestimmten Spektralbereiches bewirkt. Das optische Filter ist insbesondere als Beobachtungsfilter und/oder Detektionsfilter, Fluoreszenzbeobachtungsfilter oder Anregungsfilter einsetzbar. Das optische Filter weist insbesondere Glas oder ein kristallines Material auf. Das optische Filter kann planar oder als Filterlinse ausgebildet sein. Prinzipiell kann anstelle des optischen Filters auch ein anderes optisches Element, wie beispielsweise eine Linse, eine Blende, ein Polarisator oder ein ähnliches optisches Element, in der Filterwechselvorrichtung und/oder der Filteraufnahme angeordnet sein.

Unter "Weißlichtfilter" wird insbesondere verstanden, dass eine entsprechende Aufnahme und/oder Position frei von einem optischen Element ist oder ein optisches Element in einer entsprechenden Aufnahme und/oder Position frei von einer Filterfunktion ist, sodass das Licht und/oder Weißlicht insbesondere unverändert durchgelassen wird. Weißlicht wird von einem Weißlichtfilter insbesondere ohne Änderung seiner Lichteigenschaften, insbesondere der Wellenlängen, durchgelassen. Bei einem Weißlichtfilter kann es sich auch um ein Filter handeln, welches Nahinfrarotes Licht blockt. Ein "Weißlichtfilter" kann auch ein Filter sein, welcher das Licht filtriert, um eine Qualität des Bildes bei Beleuchtung mit weißem Licht zu verbessern. Hierfür kann z.B. ein BG39 Filter von der Firma Schott verwendet werden. Somit kann mittels eines Weißlichtfilters auch das durch einen Bildsensor und/oder eine Kamera erfasste Licht an eine Empfindlichkeitskurve und/oder eine spezifische Empfindlichkeit des menschlichen Auges angepasst werden.

Ein "Fluoreszenzbeobachtungsfilter" (auch als "Fluoreszenzfilter" bezeichnet) ist insbesondere ein optisches polychroitisches Interferenzfilter zum Trennen des emittierten Fluoreszenzlichtes von dem eingesetzten Anregungslicht. Somit blockiert das Fluoreszenzfilter die spezifische Fluoreszenzanregungsstrahlung und lässt die Fluoreszenzemissionsstrahlung entlang des optischen Strahlengangs durch. Bevorzugt blockiert das Fluoreszenzfilter vollständig das Anregungslicht, während es das Fluoreszenzemissionslicht durchlässt, welches üblicherweise eine längere Wellenlänge als das Anregungslicht aufweist. Somit handelt es sich bei einem Fluoreszenzfilter insbesondere um ein Beobachtungsfilter, welches das Anregungslicht, welches ein Fluorophor zum Leuchten bringt, herausfiltert. Dies ist vorteilhaft, da das Anregungslicht in der Regel mehrere Größenordnungen heller ist als das resultierende und/oder emittierte Fluoreszenzlicht und dieses sonst überstrahlt. Bei einem Fluoreszenzfilter kann es sich auch um ein "Blaufilter", "Rotfilter", "IR-Filter" oder "NIR-Filter" handeln.

Unter "Blaufilter" wird insbesondere ein Filter verstanden, welches das blaue Anregungslicht einer Lichtquelle herausfiltert, aber das Fluoreszenzlicht, insbesondere ein von einem Fluorophor emittiertes Fluoreszenzlicht, zumindest überwiegend durchlässt. Beispielsweise erfolgt bei der Verwendung des Fluorophor FITC (Fluorescein isothiocyanat, grünes Derivat von Fluorescein) eine Anregung mittels LED bei einer Wellenlänge von 460 nm, wobei langwelligeres Fluoreszenzlicht mit einem Maximum bei ca. 520 nm im grünen Spektralbereich vom Fluorophor emittiert wird. Um das emittierte Fluoreszenzlicht des FITC in der Bildgebung gut darstellen zu können, wird mittels der Filterwechselvorrichtung und/oder Kamera das blaue Anregungslicht herausgefiltert.

Unter "Rotfilter" wird insbesondere ein Filter verstanden, welches das rote Anregungslicht einer Lichtquelle herausfiltert, aber das Fluoreszenzlicht, insbesondere ein von einem Fluorophor emittiertes Fluoreszenzlicht, zumindest überwiegend durchlässt.

Unter "IR-Filter" wird insbesondere ein Filter verstanden, welches infrarotes Anregungslicht einer Lichtquelle herausfiltert, aber das Fluoreszenzlicht, insbesondere ein von einem Fluorophor emittiertes Fluoreszenzlicht, zumindest überwiegend durchlässt.

Unter "NIR-Filter" wird insbesondere ein Filter verstanden, welches nahinfrarotes Anregungslicht einer Lichtquelle herausfiltert, aber das Fluoreszenzlicht, insbesondere ein von einem Fluorophor emittiertes Fluoreszenzlicht, zumindest überwiegend durchlässt.

Ein "optischer Durchgang" ist insbesondere ein ausgesparter Raum in der Filterwechselvorrichtung, durch welchen Licht durchtreten kann. Ein optischer Durchgang ist insbesondere eine durchgehende Öffnung durch die rotierbare Drehplatte, dem jeweiligen Drehplattenlager, weiteren Bestandteilen und/oder des Gehäuses der Filterwechselvorrichtung. Der optische Durchgang ist insbesondere um den Mittelpunkt des Querschnittes der drehbaren Drehplatte, dem jeweiligen Drehplattenlager und/oder um die optische Achse angeordnet. Der optische Durchgang erstreckt sich insbesondere entlang der optischen Achse. In Lichtausbreitungsrichtung vor und/oder in dem optischen Durchgang kann insbesondere eine Filteraufnahme und/oder ein optisches Filter angeordnet sein. Ebenso kann der optische Durchgang bei Lichtdurchtritt frei von einem angeordneten optischen Filter und/oder einer Aufnahme sein. Prinzipiell kann der optische Durchgang sämtliche Querschnittsformen aufweisen, bevorzugt ist der optische Durchgang kreisrund im Querschnitt ausgebildet.

Eine "optische Achse" ist insbesondere eine Linie, entlang der ein Grad an Rotationssymmetrie in einem optischen System existiert. Die optische Achse ist insbesondere eine imaginäre Linie, welche einen Pfad definiert, entlang dem Licht durch die Filterwechselvorrichtung und/oder die Kamera in Richtung eines Bildsensors propagiert. Vorzugsweise läuft die optische Achse durch den Krümmungsmittelpunkt des jeweils eingeschwenkten Filters und/oder eines nachgeschalteten Linsensystems und/oder Objektivsystems. Die optische Achse kann jedoch auch durch eine Linse, ein optisches Element und/oder eines der optischen Filter gebogen und/oder gelenkt werden. Der optische Strahlengang als geometrischer Verlauf von Lichtstrahlen ist insbesondere in und/oder um die optische Achse angeordnet und verläuft entlang, konvergierend und/oder dispergierend zu der optischen Achse.

Eine "Drehplatte" (auch "Filterrad" genannt) ist insbesondere eine Platte, welche drehbar ist und mindestens eine Führungsebene auf einer Plattenfläche oder beiden Plattenflächen aufweist. Die Drehplatte ist insbesondere um ihren Drehpunkt und/oder ihre Drehachse und/oder Mittelachse drehbar. Die Drehachse der Drehplatte liegt bevorzugt in der optischen Achse. Die Drehplatte ist insbesondere relativ zu dem mindestens einen Drehplattenlager und dem mindestens einen Gehäuseteil drehbar. Die Drehplatte weist insbesondere eine Lageraussparung und/oder einen optischen Durchgang auf, welche oder welcher konzentrisch zur optischen Achse und/oder dem optischen Strahlengang angeordnet ist oder sind. Die Drehplatte kann insbesondere per Hand und/oder mittels einer Antriebseinheit und/oder einem Motor angetrieben und gedreht werden. Dazu kann beispielsweise die äußere Umfangsfläche als Antriebsfläche angetrieben werden, indem eine Antriebseinheit und/oder ein Getriebe an dieser und/oder auf diese Antriebsfläche einwirkt. Dementsprechend kann die Antriebsfläche zum Antrieb speziell ausgebildet sein, beispielsweise äußere Zahnräder aufweisen. Auch kann die Antriebsfläche für ein Drehen per Hand speziell ausgebildet sein und dazu beispielsweise nicht kreisrund, sondern mit mehreren halbkreisförmigen Aussparungen an ihrem Außenumfang versehen sein. Beispielsweise kann die Drehplatte an ihrem Außenumfang zwischen langgestreckten, halbkreisförmigen Aussparungen jeweils mehrere, insbesondere sechs, herausstehende Ecken aufweisen. Die Drehplatte ist insbesondere ein im Wesentlichen flaches, ebenes Bauteil, wobei deren gegenüberliegende Plattenflächen im Wesentlichen senkrecht zur optischen Achse ausgerichtet sind. Die Drehplatte weist insbesondere in ihrem Mittelpunkt der Plattenflächen eine Lageraussparung und/oder einen optischen Durchgang durch ihre Materialdicke auf. Die Lageraussparung dient zur drehbaren Lagerung der Drehplatte. Die Lageraussparung weist insbesondere einen größeren Durchmesser als ein Durchmesser des optischen Durchganges der Drehplatte auf. Um die Baugröße der Filterwechselvorrichtung entlang der optischen Achse möglichst gering zu halten, weist die Drehplatte insbesondere eine geringe Materialdicke auf. Die Drehplatte weist insbesondere an mindestens einer ihrer beiden Plattenflächen eine Führungsebene auf, welche flächig an der Plattenfläche angeordnet und/oder verbunden ist. Dementsprechend weist die Drehplatte im Bereich der Führungsebene oder der Führungsebenen eine größere Materialdicke als an der restlichen Plattenfläche auf. Ebenso kann die Drehplatte an ihrem äußeren Rand eine größere Materialdicke aufweisen. Zwischen dem äußeren Rand der Drehplatte und dem Mittelpunkt der Drehplatte ist insbesondere entlang der optischen Achse an der jeweiligen Plattenfläche ein Drehplattenlager bündig, jedoch zur jeweiligen Plattenfläche kontaktfrei anordenbar. Die Drehplatte und die mindestens eine Führungsebene oder die Führungsebene können mehrteilig oder einstückig ausgebildet sein. Beispielsweise können die Führungsebenen durch ein spanendes Fertigungsverfahren, wie Fräsen, aus einem plattenförmigen Werkstück zur Herstellung der Drehplatte gefertigt sein. Ebenfalls kann die Drehplatte mit einer Führungsebene oder mehreren Führungsebenen aus einem Kunststoffmaterial gegossen sein. Ebenso kann eine Führungsebene oder können mehrere Führungsebenen auf eine Rohplatte zum Ausbilden der Drehplatte stoffschlüssig verbunden, beispielsweise aufgeklebt, sein. Um die Reibungskräfte zwischen der jeweiligen Kontaktfläche der Führungsebene der Drehplatte und der Außenoberfläche des jeweiligen Führungselementes zu minimieren, weist die Drehplatte insbesondere ein Material mit einem niedrigen Reibungskoeffizienten, beispielsweise Aluminium oder ein polymeres Material, wie PTFE, auf. Aufgrund der Ausbildung der jeweiligen Führungsebene mit einer geformten, umlaufenden und geschlossenen Kontaktfläche, ist die rotierbare Drehplatte unendlich und somit mit einer beliebigen Anzahl von Umdrehungen in einer der beiden Drehrichtungen drehbar.

Eine "Führungsebene" ist insbesondere ein plattenförmiger Bereich und/oder Abschnitt der Drehplatte oder ein plattenförmiges Bauteil, welches auf einer Plattenfläche der Drehplatte angeordnet ist. Im Bereich der Führungsebene vergrößert die Führungsebene insbesondere die Materialdicke der Drehplatte entlang der optischen Achse. Eine jeweilige Führungsebene kann im Wesentlichen eine runde oder kreisrunde Querschnittsform aufweisen. Die Führungsebene weist an ihrem Außenumfang insbesondere eine Kontaktfläche auf. Bei der Kontaktfläche handelt es sich insbesondere um eine äußere Stirnfläche. Eine Führungsebene oder zwei oder mehrere Führungsebenen können insbesondere einen optischen Durchgang aufweisen und/oder um den optischen Durchgang der Filterwechselvorrichtung auf einer jeweiligen Plattenfläche angeordnet sein. Alternativ oder ergänzend kann eine Führungsebene oder können mehrere Führungsebenen auch rundumlaufend mit ihren nach innen zum optischen Durchgang ausgerichteten Kontaktflächen im äußeren Bereich der Drehplatte angeordnet sein. Somit drückt die jeweilige Kontaktfläche der Führungsebene bei Anordnung innenliegend um den optischen Durchgang von innen gegen das jeweilige Führungselement und bei Anordnung im äußeren Bereich der Drehplatte mit einer nach innen gerichteten Kontaktfläche entsprechend von außen gegen das entsprechende Führungselement. Bei Drehen der Drehplatte wird die Rotationsbewegung der Drehplatte mittels der Kontaktfläche und ihrer bewirkten Druckkraft in eine Bewegung des jeweiligen Führungselementes entsprechend der Form der Führungsebene in einer Richtung quer zur optischen Achse übertragen und somit eine Schwenkbewegung des zugehörigen Filterhalters initiiert. Die jeweilige Führungsebene kann insbesondere eine im Wesentlichen kreisförmige und/oder ringförmige Grundform aufweisen. Entlang ihres Außenumfanges kann die Führungsebene jedoch unterschiedliche Durchmesser und/oder Formabschnitte aufweisen. Jede Führungsebene kann eine Aussparung oder mehrere Aussparungen in ihrer Kontaktfläche und somit im Bereich der jeweiligen Aussparung einen geringeren Durchmesser im Querschnitt aufweisen. Zwei oder mehrere Führungsebenen einer Plattenfläche können insbesondere unterschiedliche Durchmesser und/oder Materialstärken aufweisen. Eine jeweilige Führungsebene auf beiden gegenüberliegenden Plattenflächen der Drehplatte kann prinzipiell eine gleiche Ausbildung und/oder Form aufweisen. Bevorzugt sind gleich ausgebildete Führungsebenen auf beiden Plattenflächen der Drehplatte jedoch relativ zueinander verdreht angeordnet und/oder die Filterhalter sind an den entsprechenden Gehäuseteilen zueinander verdreht angeordnet, um ein abwechselndes Einschwenken der Filteraufnahmen der Filterhalter in den optischen Durchgang der Filterwechselvorrichtung zu ermöglichen. Die jeweilige Führungsebene stellt insbesondere mit ihrer Kontaktfläche eine Kurvenbahn bereit, entlang welcher sich ein Führungselement oder mehrere Führungselemente der Filterhalter bewegen.

Ein "Drehplattenlager" ist insbesondere ein Element zum Führen der drehbaren Drehplatte innerhalb des Gehäuses der Filterwechselvorrichtung. Das Drehplattenlager und die Drehplatte sind insbesondere frei von einer axial ausgedehnten Welle. Das Drehplattenlager ist insbesondere ein speziell geformtes plattenförmiges Bauteil. Das Drehplattenlager weist insbesondere mittig einen optischen Durchgang und/oder um den optischen Durchgang ein Verbindungsteil auf. Über ein jeweiliges Verbindungsteil sind insbesondere ein distalseitiges Drehplattenlager und ein proximalseitiges Drehplattenlager miteinander verbindbar. Bei dem Verbindungsteil kann es sich insbesondere um einen Rohrabschnitt handeln. Der optische Durchgang und/oder eine Lageraussparung der Drehplatte ist insbesondere größer als der Außendurchmesser der Verbindungsteile der distalseitigen und proximalseitigen Drehplattenlager, sodass die beiden Verbindungsteile der proximalseitigen und distalseitigen Drehplattenlager innenliegend in der Lageraussparung und/oder dem optischen Durchgang der Drehplatte anordenbar sind und die Drehplatte sich um die Außenoberfläche der Verbindungsteile der Drehplattenlager drehen kann. Das distalseitige Drehplattenlager ist insbesondere fest mit dem distalseitigen Gehäuseteil und das proximalseitige Drehplattenlager ist insbesondere fest mit dem proximalseitigen Gehäuseteil verbunden. Das jeweilige Drehplattenlager ist insbesondere formschlüssig und/oder kraftschlüssig mit dem jeweiligen Gehäuseteil verbunden. Beispielsweise ist das jeweilige Drehplattenlager mit dem Gehäuseteil verschraubt. Für jedes Führungselement weist ein Drehplattenlager insbesondere eine Aussparung auf, damit das Führungselement die entsprechende Kontaktfläche der Führungsebene der Drehplatte kontaktieren kann. Die jeweilige Aussparung kann innerhalb der Plattenfläche vollständig durch die Materialdicke des Drehplattenlagers oder durch einen Einschnitt in den äußeren Rand des Drehplattenlagers ausgebildet sein. Die jeweilige Aussparung weist insbesondere eine gebogene und/oder umlaufende Form auf. Bei dem Einbringen von zwei gegenüberliegenden Aussparungen in dem äußeren Rand einer kreisrunden Platte verbleiben entsprechend zwei plattenförmige Flügel des Drehplattenlagers.

Prinzipiell ist herauszustellen, dass die Bezeichnungen "erster" und "zweiter" Filterhalter, Filter und weitere Begriffe sowie "erste" und "zweite" Führungsebene, Kontaktfläche, Plattenfläche und weitere Begriffe nur der Unterscheidung dienen. So hängt es beispielsweise beim Bewegen eines jeweiligen Führungselementes beim Drehen der Drehplatte von der Drehrichtung im Uhrzeigersinn oder entgegen des Uhrzeigersinns ab, welche Filteraufnahme des zugehörigen Filterhalters als erster in den optischen Durchgang eingeschwenkt wird.

Unter "Ausgangsposition" (auch "Ruheposition") wird insbesondere eine eingenommene oder eingehaltene Position und/oder Ausrichtung eines jeweiligen Filterhalters frei von dem optischen Durchgang verstanden. Hierbei ist das zugehörige Führungselement insbesondere in einer vorgegebenen Stelle der geformten, umlaufenden Führungsnut angeordnet, an welcher keine Druckkraft auf dieses Führungselement ausgeübt wird und somit der zugehörige Filterhalter keine Rotations- und/oder Schwenkbewegung um seine Rotationsachse ausführt. Somit ist der zugehörige Filterhalter in der Ausgangsposition in seiner Längsrichtung mit seiner nach innen ausgerichteten Außenseite insbesondere mit einem Abstand benachbart zum Außendurchmesser des optischen Durchgangs angeordnet. Damit liegt der jeweilige Filterhalter in der Ruheposition insbesondere horizontal ausgerichtet unterhalb oder oberhalb des optischen Durchgangs.

Eine "Übergangsposition" ist insbesondere eine Stelle an der Kontaktfläche einer Führungsebene und/oder mehrerer aufeinander angebrachter Führungsebenen, bei der keiner der Filterhalter in den optischen Durchgang eingeschwenkt ist.

Ein "Filterhalter" ist insbesondere ein länglich ausgebildetes Element und/oder ein Arm, welcher drehbar um seine Rotationsachse auf und/oder an der innenliegenden Seite eines Gehäuseteils angeordnet ist. Der Filterhalter kann eine tropfenförmige oder keulenförmige Form aufweisen, wobei die Filteraufnahme bevorzugt am breitesten Ende des Filterhalters angeordnet ist. Der Filterhalter weist insbesondere eine erste Bohrung in einer seiner flächigen und/oder ebenen Seiten auf, wobei in dieser Bohrung die Rotationsachse angeordnet und/oder stoffschlüssig oder kraftschlüssig verbunden, beispielsweise eingepresst, ist. Diese erste Bohrung kann durch eine Materialstärke des Filterhalters quer zu seiner Längsrichtung teilweise oder vollständig durchgehend ausgeführt sein. Eine Öffnung dieser ersten Bohrung ist insbesondere zum entsprechenden Gehäuseteil hin ausgerichtet. Die Rotationsachse des jeweiligen Filterhalters ist insbesondere endständig und/oder auf der gegenüberliegenden Seite zur Filteraufnahme des Filterhalters entlang der Längsrichtung des Filterhalters angeordnet. Die Rotationsachse kann beispielsweise als Welle ausgebildet sein. Des Weiteren weist der Filterhalter an seiner der Rotationsachse gegenüberliegenden, flächigen Seite insbesondere eine zweite Bohrung auf, in welcher das Führungselement aufgenommen und/oder befestigt ist. Diese Seite des Filterhalters ist insbesondere zu dem zugehörigen Drehplattenlager und der Drehplatte ausgerichtet ist. Der Filterhalter weist mindestens eine Filteraufnahme für ein optisches Filter auf. Bevorzugt weist jeder Filterhalter genau eine Filteraufnahme an seinem Ende gegenüberliegend zur Rotationsachse auf. Die Filterhalter weisen in ihrer Längsrichtung insbesondere gleiche und/oder unterschiedliche Längen auf. Die jeweilige Länge des Filterhalters hängt insbesondere vom jeweiligen Durchmesser der zugehörigen Führungsebene ab, welche das Führungselement des Filterhalters kontaktiert. Die Längsrichtung des Filterhalters ist insbesondere im Wesentlichen quer zur optischen Achse angeordnet.

Eine "Filteraufnahme" (auch "Aufnahme" genannt) ist insbesondere ein Hohlkörper oder Hohlraum mit einer außen teilweisen oder vollständigen Umfassung und/oder Umrandung, in welchen ein optisches Filter einsetzbar ist und welche den optischen Filter an seinem Umfang zumindest teilweise umschließt. Eine Filteraufnahme kann beispielsweise ein kurzer rohrförmiger Körper sein. Die Filteraufnahme bildet insbesondere einen Halter und/oder eine Schutzhülle für das optische Filter.

Ein "Führungselement" ist insbesondere ein Element, auf welches eine Druckkraft einer geformten, umlaufenden Kontaktfläche einer Führungsebene einwirkbar ist. Das Führungselement ist insbesondere fest oder lösbar mit dem zugehörigen Filterhalter verbunden. Das Führungselement kann insbesondere direkt mit dem Filterhalter oder indirekt über ein Verbindungselement und/oder ein Kugellager mit dem Filterhalter verbunden sein. Die Längsrichtung des Führungselementes ist insbesondere parallel zur optischen Achse ausgerichtet. Das Führungselement weist insbesondere eine derartige Länge auf, dass es sich in seiner Längsrichtung von dem Filterhalter, durch eine Aussparung des entsprechenden Drehplattenlagers bis entlang zumindest teilweise der Materialdicke derjenigen Führungsebene und/oder der Kontaktfläche der Führungsebene erstreckt, sodass eine Druckkraft der Kontaktfläche auf eine Außenfläche des Führungselementes einwirkbar ist. Hierbei muss sich das Führungselement in seiner Längsrichtung nicht zwingend über eine gesamte Materialdicke der relevanten Führungsebene und/oder der Kontaktfläche der Führungsebene erstreckt. Wenn zwei Führungsebenen mit unterschiedlichen Durchmessern auf einer Plattenfläche angeordnet sind, ist es vorteilhaft, dass das erste Führungselement, welches der oberen und/oder distaler oder proximaler angeordneten Führungsebene zugeordnet ist, zumindest geringfügig kürzer als die Länge der Materialdicke dieser Führungsebene ausgebildet ist, damit das Ende des Führungselementes nicht auf die nachfolgende darunter und/oder mittiger angeordnete Führungsebene mit einem zumindest teilweise größeren Durchmesser auftrifft und eine erhöhte Reibung verursacht. Dementsprechend können zwei und mehrere Führungselemente bei einem Kontakt mit derselben Führungsebene einer Plattenfläche oder jeweils einer Führungsebene mit der gleichen Anordnung und Materialdicke auf beiden Plattenflächen die gleiche Länge in Längsrichtung aufweisen. Ebenso können zwei und mehrere Führungselemente bei Kontaktierung mit jeweils aufeinander und/oder nacheinander entlang der optischen Achse angeordnete Führungsebenen unterschiedliche Längen aufweisen, um jeweils die vorgesehene Kontaktfläche einer der Führungsebenen zu kontaktieren. Ein Führungselement ist insbesondere stabförmig und/oder zylinderförmig. Bei einem Führungselement kann es sich insbesondere um einen Führungsstift oder einen Führungsbolzen handeln. Alternativ oder ergänzend können die Filterhalter mit ihren Rotationsachsen auch auf verschiedenen Ebenen des Gehäuseteils befestigt sein, sodass dadurch gleiche lange Führungselemente unterschiedlich weit an die Kontaktflächen geführt sind. Beim Drehen der Drehplatte läuft das zugehörige Führungselement zumindest teilweise außen um den Umriss der Führungsebene und somit entlang der geformten, umlaufenden Kontaktfläche der Führungsebene.

Eine "Gehäuseteil" ist insbesondere ein Bestandteil der Filterwechselvorrichtung, auf und/oder an welchem die mindestens zwei schwenkbaren Filterhalter drehbar befestigt sind. Das Gehäuseteil ist insbesondere frei von einer Führungsebene. Bei dem Gehäuseteil kann es sich um ein distales oder proximales Gehäuseteil und/oder um einen distalen oder proximalen Gehäusedeckel der Filterwechselvorrichtung handeln. Bei dem Gehäuseteil kann es sich auch um eine Grundplatte innenliegend im Gehäuse handeln. Zwischen dem proximalen und dem distalen Gehäuseteilen und/oder -deckeln ist insbesondere die Drehplatte rotierbar derart angeordnet, dass die Außenoberfläche der Drehplatte zumindest an ihrem Außenumfang von außen frei zugängig ist. Auch kann die Drehplatte einen größeren Durchmesser aufweisen als ein Gehäuseteil oder beide Gehäuseteile. Die beiden proximalen und distalen Gehäuseteile sind insbesondere nicht direkt miteinander, sondern innenliegend im Gehäuse über die beiden Drehplattenlager miteinander, verbunden. Das jeweilige Gehäuseteil weist innenliegend insbesondere für jede Rotationsachse eine Bohrung auf.

Eine "Kamera" (auch "Kamerakopf" genannt) ist insbesondere ein Gerät zum Empfangen von Bildlicht entlang einer optischen Achse von einem Endoskop und zum Fokussieren des empfangenen Bildlichtes auf mindestens einem Bildsensor. Neben dem mindestens einen Bildsensor kann die Kamera insbesondere eine Blende oder ein Fenster zum Durchlassen des empfangenen Bildlichtes und ein Linsensystem zum Fokussieren des Bildlichtes auf den mindestens einen Bildsensor aufweisen. Die durch mindestens einen Bildsensor aufgenommenen Bilddaten sind insbesondere elektronisch vom Kamerakopf weiter an ein Anzeigesystem und/oder an eine Bildverarbeitungseinheit übertragbar, um das endoskopische Bild für den Benutzer darzustellen. Die Kamera kann Mittel zum Erkennen des verbundenen Endoskopes und zum Verarbeiten von Algorithmen aufweisen. Ein Verbinder zum Verbinden eines Endoskops mit der Kamera kann am distalen Ende des Endoskops und/oder dem proximalen Ende des Kamerakopfs angeordnet sein. Auch kann die erfindungsgemäße Filterwechselvorrichtung selbst als Verbinder zum Verbinden eines Endoskopes mit einer Kamera ausgebildet sein.

Ein "Endoskop" ist insbesondere ein medizinisches oder industrielles Gerät zur endoskopischen Untersuchung und Betrachtung einer menschlichen oder tierischen Körperhöhle und/oder eines industriellen Hohlraums, wie einem Rohr. Das Endoskop weist insbesondere ein Handstück, einen Schaft, eine Lichtquelle, einen Lichtleiter, einen Sensor und/oder eine Kamera auf. Bei dem Endoskop handelt es sich insbesondere um ein Videoendoskop, welches eine digitale Bildaufnahme und Bildübertragung und somit eine integrierte oder verbindbare Kamera aufweist. Neben human- und tiermedizinischen Anwendungen kann ein Endoskop und/oder Videoendoskop auch zu industriellen Zwecken, beispielsweise zur Sichtprüfung in schwer zugänglichen Hohlräumen, eingesetzt werden. Bei industriellen Anwendungen wird ein Endoskop häufig als Boroskop bezeichnet.

Ein "Bildsensor" ist insbesondere ein lichtempfindliches elektronisches Bauelement, welches auf einem inneren Photoeffekt beruht. Mittels des Bildsensors wird insbesondere ein Bild oder werden mehrere Bilder aus dem Betrachtungsbereich der Bildgebungsvorrichtung aufgezeichnet und in elektronische Signale umgewandelt. Der Bildsensor weist eine Sensorebene in der Bildebene des optischen Systems, eines Linsensystems und/oder des Objektivs auf. Bei einem elektronischen Bildsensor kann es sich insbesondere um einen CCD-Sensor (charge-coupled device) oder um einen CMOS-Sensor (complementary metal oxide-semiconductor) handeln.

Unter "distalseitig" und "distal" wird insbesondere eine benutzerferne Anordnung und/oder ein entsprechendes Ende oder ein Abschnitt verstanden. Dementsprechend ist bei einem Verbinder der Filterwechselvorrichtung mit einem Endoskop das Endoskop distalseitig angeordnet. Dementsprechend wird unter "proximalseitig" oder "proximal" eine benutzernahe Anordnung oder ein entsprechendes Ende oder ein Abschnitt verstanden. Bei Verbindung der Filterwechselvorrichtung mit einer Kamera und/oder einem Kamerakopf ist entsprechend die Kamera und/oder der Kamerakopf proximalseitig von der Filterwechselvorrichtung angeordnet.

In einer weiteren Ausführungsform weist die Filterwechselvorrichtung zugeordnet zu jedem Filterhalter mindestens jeweils ein federndes Element auf, wobei mittels des mindestens einen federnden Elementes derart auf den jeweiligen Filterhalter einwirkbar ist, dass eine Federkraft des mindestens einen federnden Elementes entgegen der Druckkraft der geformten, umlaufenden Kontaktfläche gerichtet ist.

Somit wird eine Filterwechselvorrichtung mit gefederten, insbesondere torsionsgefederten, Filterhaltern bereitgestellt. Mittels mindestens einem federnden Element oder zwei oder mehrerer federnder Elemente ist gewährleistet, dass das jeweilige Führungselement des Filterhalters nicht den Kontakt zur Kontaktfläche der Führungsebene verliert. Hierzu kann das federnde Element an dem jeweiligen Filterhalter und einer Innenseite des Gehäuseteiles angreifen und dadurch eine Federkraft auf den Filterhalter auswirken, um das Führungselement gegen die Kontaktfläche zu drücken. Bevorzugt ist die Federkraft des jeweiligen Federelementes der Druckkraft der geformten, umlaufenden Kontaktfläche im Wesentlichen entgegengerichtet. Unter im Wesentlichen entgegengerichtet wird hierbei verstanden, dass die Federkraft und die Druckkraft nicht exakt aus gegenläufigen Richtungen aufeinander ausgerichtet sein müssen. Dadurch, dass durch die Änderung der umlaufenden Form der Kontaktfläche entlang des Umrisses der Kontaktfläche sich die ausgeübte Druckkraft entsprechend der Formänderung in ihrer Stärke und/oder Richtung ändert, muss die entgegengerichtete Federkraft nicht stets genau entgegengerichtet wirken, jedoch ausreichend sein, um zu gewährleisten, dass das jeweilige Führungselement an die Kontaktfläche gedrückt wird.

Dadurch, dass jeder Filterhalter mit mindestens einem federnden Element gefedert ist, wird nicht eine zweite Seitenwand gegenüber der geformten, umlaufenden Kontaktfläche und/oder eine Führungsnut benötigt, um die Führungselemente definiert entlang der geformten, umlaufenden Kontaktfläche zu führen. Stattdessen werden die Führungselemente durch Aufbringen einer jeweiligen Federkraft mittels eines zugehörigen federnden Elementes gegen die jeweilige geformte, umlaufende Kontaktfläche der Führungsebenen gedrückt. Dadurch wird die Anzahl der Reibflächen minimiert und gegenüber einer Ausgestaltung mit einer Führungsnut halbiert. Folglich wird sowohl die Reibung an der jeweiligen geformten, umlaufenden Kontaktfläche der Führungsebene als auch an der Außenoberfläche des jeweiligen Führungselementes minimiert. Neben dem Verschleiß wird durch das federnde Element auch ein Verklemmen des jeweiligen Führungselementes verhindert, da das federnde Element auf die Außenseite des Filterhalters, jedoch nicht auf das Führungselement selbst einwirkt und somit das Führungselement nur einseitig durch die Kontaktfläche begrenzt ist.

Selbst wenn bei einem Langzeitbetrieb ein Verschleiß an der geformten, umlaufenden Kontaktfläche und/oder dem jeweiligen Führungselement der Filterwechselvorrichtung auftritt, so wird trotz dadurch auftretenden Spiel aufgrund der Federkraft des federnden Elementes das jeweilige Führungselement optimal an die geformte, umlaufende Kontaktfläche gedrückt, sodass dennoch die vordefinierte Ausschwenk- oder Einschwenkbewegung und/oder -position sowie eine Ausgangs- und/oder Ruheposition des jeweiligen Filterhalters eingehalten wird. Über die Rotationsachse des jeweiligen Filterhalters wird zwar eine Schwenkbewegung ermöglicht, das genaue Einhalten der gewünschten Bewegung und/oder Position des jeweiligen Filterhalters wird jedoch im Zusammenwirken der Druckkraft der geformten, umlaufenden Kontaktfläche und der Federkraft des mindestens einen federnden Elementes realisiert und dadurch das präzise Einnehmen und/oder Einhalten der gewünschten Bewegung und/oder Position gewährleistet. Durch das mindestens eine federnde Element ist gleichzeitig ein Einschnappen des jeweiligen Filterhalters in der Einschwenkposition oder der Ausgangsposition ermöglicht. Insbesondere durch zwei federnde Elemente je Filterhalter kann der zugehörige Filterhalter in der gewünschten Position und im eingeschwenkten Zustand konzentrisch zum Strahlengang gehalten werden und gleichzeitig eine Einrastfunktion realisiert werden.

Wenn die jeweiligen Führungsebenen mit den Kontaktflächen innenliegend und/oder um den optischen Durchgang herum angeordnet sind und somit das jeweilige Führungselement nach außen drücken, drückt das mindestens eine federnde Element oder die federnden Elemente von außen gegen den jeweiligen Filterhalter in Richtung auf den optischen Durchgang und/oder Strahlengang. In einer Alternative des Mechanismus kann die Wirkweise des mindestens einen federnden Elementes oder der federnden Elemente auch umgekehrt sein. In diesem Fall ist die jeweilige Führungsebene außen am Umfang der Drehplatte angeordnet und drückt über die Führungselemente die Filterhalter nach innen in Richtung auf den optischen Durchgang und/oder Strahlengang. Dementsprechend ist das mindestens eine federnde Element oder sind die federnden Elemente innenliegend um die Mitte und/oder den optischen Strahlengang angeordnet und drücken mittels der Federkraft den jeweiligen Filterhalter nach außen vom optischen Durchgang und/oder Strahlengang weg.

Ein "federndes Element" ist insbesondere ein technisches Bauteil, welches elastisch verformbar ist. Bei der elastischen Verformung des federnden Elementes handelt es sich insbesondere um eine Biegung und/oder eine Torsion. Das federnde Element weist insbesondere Metall und/oder Kunststoff auf. Bevorzugt weist das federnde Element Stahl auf. Bei dem federnden Element kann es sich beispielsweise um eine Schenkelfeder handeln. Eine Schenkelfeder ist insbesondere eine schraubenförmig gewickelte Drahtfeder mit abstehenden geraden Enden und/oder Schenkeln. Hierbei kann ein Schenkel auf einen Filterhalter einwirken und der andere Schenkel am Gehäuseteil befestigt sein. Die Schenkel dienen insbesondere zur Einleitung des den Draht biegenden Drehmoments. Der schraubenförmig gewickelte Teil der Schenkelfeder kann insbesondere auf einem eingesteckten Zylinder und/oder Pin geführt sein. Bei einer Schenkelfeder kann es sich auch um eine Torsionsfeder handeln. Bei einer Torsionsfeder kann es sich um eine gerade, gestreckte Torsionsfeder, wie eine Drehstabfeder, oder um eine gewundene Torsionsfeder, wie eine Schraubenfeder, handeln. Der Schenkel der Torsionsfeder, welcher gegen den Filterhalter drückt, kann insbesondere in eine Nut entlang des Außenumrisses des Filterhalters einwirken. Durch die Anzahl der Schraubwindungen der Schenkelfeder und/oder Torsionsfeder kann insbesondere die Federkraft vorgegeben werden.

Um die Anzahl der Filterhalter weiter zu erhöhen und dabei eine Bewegungseinschränkung und/oder Kollision von mehr als zwei schwenkbaren Filterhaltern in einer Ebene an der ersten Plattenfläche zu vermeiden, kann die Filterwechselvorrichtung mindestens ein zweites Gehäuseteil, mindestens ein zweites Drehplattenlager und mindestens einen weiteren schwenkbaren Filterhalter, insbesondere zwei weitere schwenkbare Filterhalter, angeordnet an dem zweiten Gehäuseteil aufweisen, wobei das zweite Drehplattenlager zwischen dem zweiten Gehäuseteil und einer zweiten Plattenfläche der Drehplatte angeordnet ist und die Drehplatte auf ihrer zweiten Plattenfläche mindestens eine erste Führungsebene mit einer geformten, umlaufenden Kontaktfläche an einem Umfang aufweist.

Somit kann bei einer weiterhin kompakten Baugröße der Filterwechselvorrichtung ein Filterwechsel zwischen mindestens drei und bevorzugt vier schwenkbaren Filterhaltern mit entsprechenden Filtern realisiert werden.

Bevorzugt sind die Filterhalter und/oder die Führungsebenen auf beiden Seiten der Drehplatte relativ zueinander versetzt und/oder verdreht in der Filterwechselvorrichtung angeordnet, um ein abwechselndes Einschwenken der Filter mittels der Filterhalter zu ermöglichen.

Dadurch, dass die Filterhalter, ihre Lagerung, die Drehplattenlager und die Führungsebenen symmetrisch aufgebaut sind, kann der Filterwechselmechanismus im Links- und Rechtslauf betrieben werden. Zudem wird für den Anwender dadurch vorteilhaft sichergestellt, dass die Bewegungen der Filterhalter bei Links- und Rechtslauf identisch und nur gespiegelt ablaufen.

In einer weiteren Ausführungsform der Filterwechselvorrichtung weist oder weisen die erste Plattenfläche und/oder die zweite Plattenfläche eine zweite Führungsebene mit einer geformten, umlaufenden Kontaktfläche auf, sodass ein erstes Führungselement entlang der ersten Kontaktfläche der ersten Führungsebene und ein zweites Führungselement entlang der zweiten Kontaktfläche der zweiten Führungsebene jeweils der ersten Plattenfläche und/oder der zweiten Plattenfläche bewegbar sind.

Dadurch können zwei Filterhalter mittels zweier Kontaktflächen zweier Führungsebenen separat und individuell mittels ihrer Führungselemente bewegt und somit ein- und/oder ausgeschwenkt oder in einer Ausgangsposition gehalten werden. Es ist besonders vorteilhaft, dass die jeweilige Führungsebene spezifisch im Hinblick auf die unterschiedlich häufige Verwendung eines Filterhalters und/oder Filters in der Filterwechselvorrichtung ausgebildet sein kann.

Um die Schwenkbewegung und/oder Position eines jeden Filterhalters und/oder seines zugehörigen Filters individuell einzustellen, können die erste Führungsebene und die zweite Führungsebene der ersten Plattenfläche und/oder der zweiten Plattenfläche unterschiedliche Durchmesser und/oder unterschiedlich geformte, umlaufende Kontaktflächen aufweisen.

In einer weiteren Ausführungsform der Filterwechselvorrichtung weisen die Filterhalter eine gleiche Länge und/oder eine unterschiedliche Länge auf.

Somit können sowohl die Filterhalter einer Plattenfläche zueinander eine gleiche Länge oder eine unterschiedliche Länge sowie jeweils zu den Filterhaltern der gegenüberliegenden Plattenfläche eine gleiche Länge und/oder eine unterschiedliche Länge aufweisen.

Um eine definierte Position für ein Einschwenken des jeweiligen Filterhalters in den optischen Durchgang sowie einen Ablauf der diesbezüglichen Einschwenkbewegung vorzugeben, können die erste Führungsebene und/oder die zweite Führungsebene jeweils mindestens eine Aussparung in einer Kontaktfläche aufweisen, sodass bei Anordnung eines Führungselementes in der Aussparung die Filteraufnahme des zugehörigen Filterhalters in den optischen Durchgang der Drehplatte präzise einschwenkbar ist.

Somit kann durch die Form und/oder Tiefe der Aussparung in der jeweiligen Kontaktfläche gezielt der Ablauf der Einschwenkbewegung und der zeitliche Verlauf zum Erreichen der Einschwenkposition in Abhängigkeit von der Drehgeschwindigkeit vorgegeben werden.

Um einen Filterhalter und somit ein bestimmtes Filter mehrmals bei einer Umdrehung der Drehplatte einzuschwenken, können in einer Kontaktfläche zwei, drei, vier oder optional weitere Aussparungen eingebracht sein, sodass unabhängig von der Drehrichtung der Halter das zugehörige Filter mehrmals eingeschwenkt wird. Bevorzugt sind die Aussparungen in einer Kontaktfläche zum Einschwenken desselben Filterhalters gleichartig ausgeführt.

Eine "Aussparung" ist insbesondere ein ausgesparter Raum und/oder eine Öffnung teilweise oder vollständig in der Kontaktfläche und/oder der flächigen Seite oder Seiten einer Führungsebene. Eine Aussparung ist insbesondere ein Einschnitt in die Kontaktfläche und somit in die Außenfläche einer Führungsebene. Im Bereich der Aussparung weist die Führungsebene im Querschnitt insbesondere einen geringeren Außendurchmesser auf. Somit bewegt sich das zugehörige Führungselement entlang der geformten Aussparung der Kontaktfläche, sodass bei einer Position in der Aussparung, bei welcher der geringste Durchmesser der Führungsebene im Querschnitt vorliegt, der zugehörige Filterhalter mit seiner Filteraufnahme in den optischen Durchgang eingeschwenkt ist. Die Aussparung ist insbesondere spezifisch geformt und kann beispielsweise konisch zulaufend auf den optischen Durchgang ausgebildet sein. Bevorzugt weist die Aussparung gerade und/oder gebogene Abschnitte auf. Beispielsweise kann die Aussparung durch mehrere nacheinander folgende Kreisbogensegmente ausgebildet sein. Im Falle von zwei aneinander angeordneten Führungsebenen auf einer Plattenfläche kann neben einer stärker ausgeformten Aussparung und somit einem größeren Einschnitt in Richtung auf den optischen Durchgang der direkt an der Plattenfläche anliegenden Führungsebene die darüber und/oder distalseitig oder proximalseitig angeordnete Führungsebene an derselben Position ebenfalls eine geringfügiger ausgebildete Aussparung für das Führungselement der direkt auf der Plattenfläche angeordneten Führungsebene aufweisen, um dieses Führungselement gezielt beim Aufbringen der Druckkraft über einen Großteil seiner Länge zu führen und/oder positionieren. Daneben weist die darüber und/oder distalseitig oder proximalseitig angeordnete Führungsebene eine stärker ausgebildete Aussparung für das dieser Führungsebene zugeordnete Führungselement auf.

In einer weiteren Ausführungsform der Filterwechselvorrichtung ist oder sind die erste Kontaktfläche der ersten Führungsebene, die zweite Kontaktfläche der zweiten Führungsebene, die mindestens eine Aussparung oder die Aussparungen symmetrisch ausgebildet und/oder angeordnet.

Durch die symmetrische Ausbildung ist die zeitliche Abfolge und Reihenfolge der nacheinander eingeschwenkten Filter unabhängig von der Drehrichtung der Drehplatte.

Um unterschiedliche Filter unterschiedlich häufig bei einer Umdrehung der Drehplatte einzuschwenken, können die erste Plattenfläche und die zweite Plattenfläche jeweils eine zweite Führungsebene mit jeweils einer Aussparung zum Einschwenken eines Filterhalters mit einem ersten optischen Filter aufweisen, und eine der beiden Plattenfläche kann eine erste Führungsebene mit vier gleichmäßig beabstandeten Aussparungen zum Einschwenken eines Filterhalters mit einem zweiten optischen Filter aufweisen, sodass jeweils eines der ersten optischen Filter und das zweite optische Filter nacheinander abwechselnd bei einer Umdrehung der Drehplatte einschwenkbar sind.

Im Falle, dass es sich bei dem ersten optischen Filter um ein Fluoreszenzfilter und dem zweiten optischen Filter um ein Weißlichtfilter handelt, ist das üblicherweise am häufigsten genutzte Weißlichtfilter abwechselnd mit einem Fluoreszenzfilter des jeweiligen Filterhalters zugeordnet zur zweiten Führungsebene der ersten Plattenfläche und der zweiten Plattenfläche einschwenkbar. Dadurch, dass das Weißlichtfilter mittels des zugehörigen Filterhalters zwischen jeder Einschwenkposition eines entsprechenden Fluoreszenzfilters eingeschwenkt wird, kann der Anwender schnell zwischen einem gewünschten Fluoreszenzmodus und dem Weißlichtmodus wechseln. Durch das Zwischenschalten des Weißlichtfilters wird dem Anwender ein Wechsel zwischen gleichartigen oder andersartigen Fluoreszenzfiltern eindeutig optisch angezeigt. Somit wird das Risiko vermindert, dass unbeabsichtigt in einem falschen Beobachtungsmodus gearbeitet wird.

Diesbezüglich ist herauszustellen, dass es sich bei dem ersten optischen Filter und dem zweiten optischen Filter prinzipiell um jede Art von Filtern handeln kann und auch die Anzahl von Aussparungen in jeder Kontaktfläche einer jeweiligen Führungsebene spezifisch gewählt und unterschiedlich zur Anzahl der Aussparungen einer anderen Führungsebene ausgebildet sein kann, um gezielt einen Filterhalter oder mehrere Filterhalter mehr als einmal pro Umdrehung der Drehplatte einzuschwenken.

Dadurch, dass zwei Filterhalter zugeordnet einer Plattenfläche unterschiedliche Längen aufweisen, kommt jeweils nur ein Führungselement mit einer Kontaktfläche der ersten Führungsebene oder der zweiten Führungsebene in Eingriff und somit können zwei Filterhalter unabhängig voneinander an der einen Plattenfläche bewegt werden.

In einer weiteren Ausführungsform der Filterwechselvorrichtung sind die erste Führungsebene und/oder die zweite Führungsebene der ersten Plattenfläche und/oder der zweiten Plattenfläche derart ausgebildet, dass in einer Übergangsposition der optische Durchgang frei von einem eingeschwenkten Filterhalter ist.

Somit weist eine jeweilige Führungsebene eine Position auf, bei welcher sich kein Filter im Strahlengang befindet. Diese Übergangsposition ist insbesondere dann erreicht, wenn ein zweites Führungselement genau gegenüberliegend zu einer Aussparung an der Kontaktfläche derselben Führungsebene angeordnet ist, wobei an dieser Übergangsposition keine Aussparung vorliegt und/oder ein maximaler Durchmesser dieser Führungsebene vorliegt und die erste Führungsebene an dieser Position ebenfalls frei von einer Aussparung ist.

Um beide Hälften des Schwenkmechanismus und somit das proximale Gehäuseteil und das distale Gehäuseteil zu verbinden, kann das erste Drehplattenlager ein erstes Verbindungselement und das zweite Drehplattenlager ein zweites Verbindungselement aufweisen, wobei das erste Verbindungselement und das zweite Verbindungselement formschlüssig und/oder kraftschlüssig miteinander verbindbar sind.

Unter "formschlüssig" wird insbesondere verstanden, dass die beiden Verbindungselemente zum Ausbilden der Verbindung ineinandergreifen. Bei einer formschlüssigen Verbindung blockiert insbesondere eines der beiden Verbindungselement die Bewegung des anderen Verbindungselementes. Somit liegt insbesondere eine Sperrung zumindest in einer Richtung vor. Bei der formschlüssigen Verbindung kann es sich beispielsweise um eine Nut-Feder-Verbindung, eine Passfeder, eine Verzahnung oder eine Drehblockierung handeln.

Unter "kraftschlüssig" wird insbesondere eine Verbindung verstanden, bei welcher eine Normalkraft auf die miteinander verbindenden Flächen der beiden Verbindungselemente besteht und ihre gegenseitige Verschiebung verhindert ist, solange die durch eine Haftreibung bewirkte Gegenkraft nicht überschritten wird. Bei einer kraftschlüssigen Verbindung kann es sich insbesondere um eine Schraubverbindung oder eine Klemmverbindung handeln.

Hierbei kann die Verbindungsstelle der beiden Verbindungselemente die Lagerstelle für die Drehplatte ausbilden. Beispielsweise bei einer Ausbildung der Verbindungselemente als Rohrabschnitte kann die Drehplatte mit ihrer mittigen Lageraussparung und/oder ihrem optischen Durchgang sich außen um die Außenoberfläche der rohrförmigen Verbindungsstelle drehen.

In einer weiteren Ausführungsform der Filterwechselvorrichtung sind das erste Gehäuseteil und das zweite Gehäuseteil in ihrem Inneren miteinander verbunden, sodass die Drehplatte über ihren gesamten Umfang von außen frei kontaktierbar und/oder drehbar ist.

Dadurch hat der Anwender über den gesamten Außenumfang vollen Zugriff auf die Drehplatte und es gibt keine Befestigungselemente, welche das Angreifen und/oder Kontaktieren des Außenumfanges der Drehplatte behindern. Folglich kann die Drehplatte von außen optimal manuell und/oder motorisch angetrieben werden. Somit ist die Drehplatte frei zugänglich und handhabbar.

Um die Filterwechselvorrichtung kontaktlos zu betreiben, können das erste Gehäuseteil und das zweite Gehäuseteil ein hermetisch dichtes Gehäuse ausbilden, wobei die Drehplatte innerhalb des Gehäuses angeordnet ist und mindestens ein Gegenmagnetelement aufweist, sodass ein Drehen der Drehplatte mittels einer außerhalb des Gehäuses zugeordneten Magneteinrichtung bewirkbar ist.

Dadurch kann eine leichtere Reinigbarkeit und Autoklavierbarkeit der Filterwechselvorrichtung realisiert sein.

In einem weiteren Aspekt der Erfindung wird die Aufgabe gelöst durch einen Kamerakopf für ein Endoskop, wobei der Kamerakopf einen Bildsensor, eine Öffnung zum Empfangen von Licht eines Bildes entlang eines optischen Pfades und ein optisches Linsensystem zum Fokussieren des Lichtes auf den Bildsensor aufweist, wobei der Kamerakopf zumindest eine zuvor beschriebene Filterwechselvorrichtung aufweist.

Somit wird ein Kamerakopf bereitgestellt, an dem oder in dem eine kompakte, platzsparend ausgebildete Filterwechselvorrichtung angeordnet ist. Im Falle der Anordnung zumindest einer Filterwechselvorrichtung direkt in dem Kamerakopf kann der Kamerakopf lösbar mit verschiedenen Arten von Endoskopen verbunden werden. Beispielsweise kann die Filterwechselvorrichtung in dem Kamerakopf integriert werden, indem diese anstelle eines Bajonett-Verschlusses verbaut ist. Dadurch kann eine Verlängerung des Strahlenganges minimiert werden. Selbstverständlich kann der Kamerakopf auch zwei oder mehrere Filterwechselvorrichtungen in Reihe in einem optischen Pfad und/oder entlang der optischen Achse aufweisen, falls die gleichzeitige Anwendung von zwei oder mehreren Filtern im Strahlengang, insbesondere in multispektralen Bildgebungen und/oder in einer breiten Anwendung von verschiedenen Fluorophoren in der Fluoreszenz-Bildgebung, gewünscht ist.

Um eine derzeitig verwendete Filterkonfiguration oder diese für gewünschte unterschiedliche Beobachtungsmodi anzupassen, kann der Kamerakopf eine Detektionseinheit zum Detektieren einer Identifikation des jeweiligen optischen Filters in dem optischen Pfad aufweisen. Ebenso kann der Kamerakopf eine Kontrolleinheit zum Anpassen der Drehgeschwindigkeit der Drehplatte und zum Überprüfen und/oder Anpassen des jeweiligen optischen Filters angeordnet im Strahlengang entsprechend des jeweils gewählten Betriebsmodus aufweisen.

In einem weiteren Aspekt der Erfindung wird die Aufgabe gelöst durch einen Nachrüstsatz zum Nachrüsten eines Kamerakopfes und/oder eines Endoskopes, wobei der Nachrüstsatz zumindest eine zuvor beschriebene Filterwechselvorrichtung aufweist, sodass die Filterwechselvorrichtung zwischen einem proximalen Ende des Endoskops und einem distalen Ende des Kamerakopfes anordenbar ist.

Somit wird ein Nachrüstsatz (auch als "Adapter" bezeichnet) mit mindestens einer Filterwechselvorrichtung bereitgestellt, welcher gleichzeitig als Verbinder zwischen einem bereits existierenden Endoskop und einem existierenden Kamerakopf als auch zum Ermöglichen von unterschiedlichen Beobachtungsmodi dient. Zusätzlich kann der Nachrüstsatz auch zwei oder mehrere Filterwechselvorrichtungen aufweisen, welche in Reihe zwischen dem Endoskop und dem Kamerakopf angeordnet sind oder eine Filterwechselvorrichtung kann durch eine andere Filterwechselvorrichtung zum Ermöglichen von verschiedenen Anwendungen und/oder Beobachtungsoptionen ersetzt werden.

Im Weiteren wird die Erfindung anhand von Ausführungsbeispielen näher erläutert. Es zeigen
Figur 1 eine schematische dreidimensionale Ausschnittsansicht eines Endoskopsystems mit einem Endoskop, einem Filterwechsler und einem Kamerakopf,
Figur 2 eine dreidimensionale Darstellung eines Filterwechslers in Seitenansicht mit einer Endoskopaufnahme und einer Kameraaufnahme,
Figur 3 eine Explosionsdarstellung von Bestandteilen der Filterwechselvorrichtung,
Figur 4 eine stark schematische Darstellung in Draufsicht auf eine proximale Seite eines Filterrades,
Figur 5 eine stark schematische Darstellung in Draufsicht auf eine distale Seite des Filterrades,
Figur 6 eine stark schematische, dreidimensionale Darstellung der proximalen Seite des Filterrades,
Figur 7 eine stark schematische Darstellung eines proximalen Drehscheibenlagers,
Figur 8 eine stark schematische Darstellung eines distalen Drehscheibenlagers, und
Figur 9 eine stark schematische Darstellung einer Alternative eines proximalen Drehscheibenlagers mit innenliegenden Aussparungen.

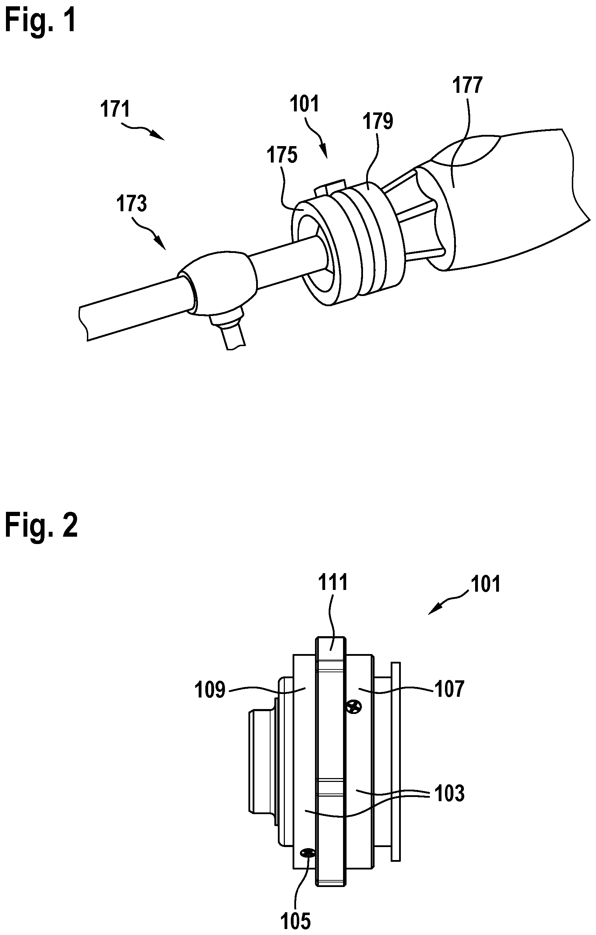

Ein Endoskopsystem 171 weist einen Kamerakopf 177, einen Filterwechsler 101 und ein Endoskop 173 auf. Der Filterwechsler 101 ist mittels einer Kameraaufnahme 179 an dem Kamerakopf 177 und mittels einer Endoskopaufnahme 175 mit dem Endoskop 173 verbunden (Figur 1 und 2).

Der Filterwechsler 101 weist ein Gehäuse 103 mit einer distalen Deckelhälfte 107 und einer proximalen Deckelhälfte 109 auf, zwischen denen ein Filterrad 111 angeordnet ist. Jeweils mittels nach innen gerichteten Schrauben 105 ist die distale Deckelhälfte 107 mit einem distalen Drehscheibenlager 130 und ist die proximale Deckelhälfte 109 mit einem proximalen Drehscheibenlager 133 innen im Gehäuse 103 verbunden sind. Dadurch ist das Filterrad 111 zwischen der distalen Deckelhälfte 107 und der proximalen Deckelhälfte 109 von außen frei kontaktierbar und drehbar.

Die distale Deckelhälfte 107 und die proximale Deckelhälfte 109 weisen jeweils konzentrisch zu einer optischen Achse 129 einen optischen Durchgang 127 auf. Zwischen einer Innenseite der proximalen Deckelhälfte 109 und einer proximalen Radfläche 115 des Filterrades 111 ist das proximale Drehscheibenlager 133 und zwischen einer distalen Radfläche 113 und einer Innenfläche der distalen Deckelhälfte 107 ist das distale Drehscheibenlager 130 angeordnet. An der Innenseite der proximalen Deckelhälfte 109 ist ein erster Filterhalter 141 und ein zweiter, langer Filterhalter 142 sowie an der Innenseite der distalen Deckelhälfte 107 ein dritter Filterhalter 143 und ein vierter Filterhalter 144 befestigt. Die Filterhalter 141, 142, 143 und 144 sind jeweils tropfenförmig ausgebildet und weisen an ihrem schmaleren Ende eine Bohrung 145 auf. In der jeweiligen Bohrung 145 ist jeweils eine Rotationsachse 149 aufgenommen, welche jeweils an der Innenseite der distalen Deckelhälfte 107 oder der proximalen Deckelhälfte 109 befestigt ist. Gegenüber der jeweiligen Bohrung 145 weist der erste Filterhalter 141 eine erste Filteraufnahme 151, der zweite Filterhalter 142 eine zweite Filteraufnahme 152, der dritte Filterhalter 143 eine dritte Filteraufnahme 153 und der vierte Filterhalter 144 eine vierte Filteraufnahme 154 für jeweils ein optisches Filter auf. Zwischen der Bohrung 145 und der jeweiligen Filteraufnahme 151, 152, 153, 154 weist jeder Filterhalter 141, 142, 143, 144 einen langen Führungsbolzen 155 oder einen kurzen Führungsbolzen 157 auf.

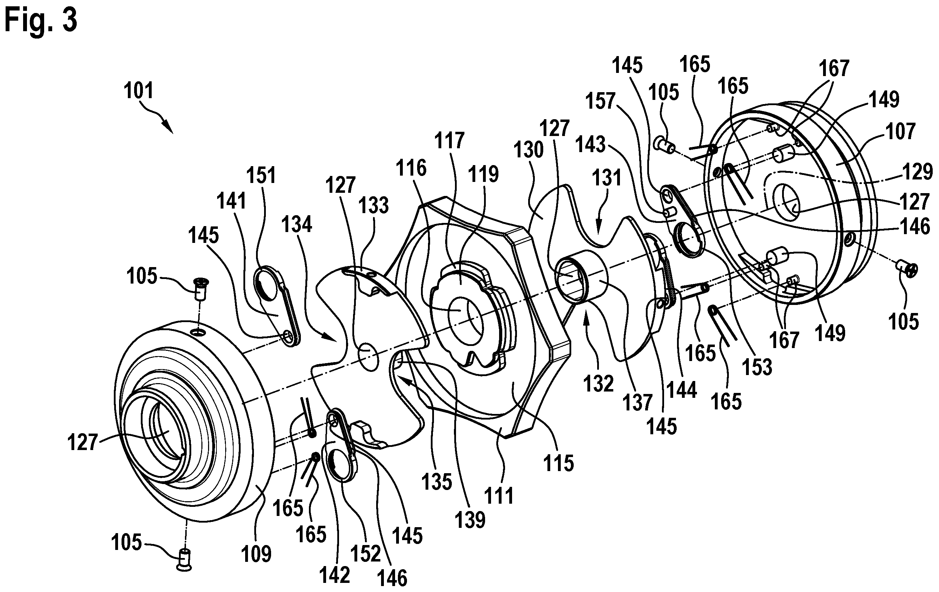

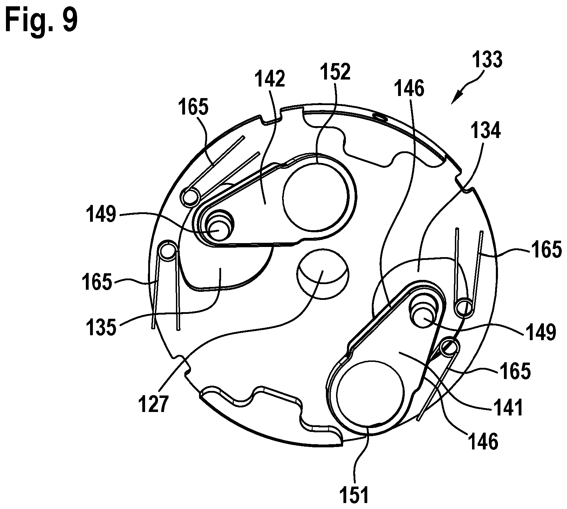

Beidseitig jeder Rotationsachse 149 sind jeweils zwei Torsionsfedern 165 angeordnet, wobei ein Schenkel jeder Torsionsfeder 165 innenliegend mit der Innenseite der entsprechenden distalen Deckelhälfte 107 oder proximalen Deckelhälfte 109 verbunden ist und der andere Schenkel in eine Nut 146 eingreift, welche beidseitig umlaufend um den konischen Abschnitt und somit um die Rotationsachse 149 jedes Filterhalters 141, 142, 143, 144 in seine jeweilige Außenfläche eingebracht ist. Jede Torsionsfeder 165 ist mit ihrer endständigen Wicklung mittels eines Haltepins 167 an der Innenoberfläche der distalen Deckelhälfte 107 oder der proximalen Deckelhälfte 109 befestigt (Figur 3 und Figur 9).

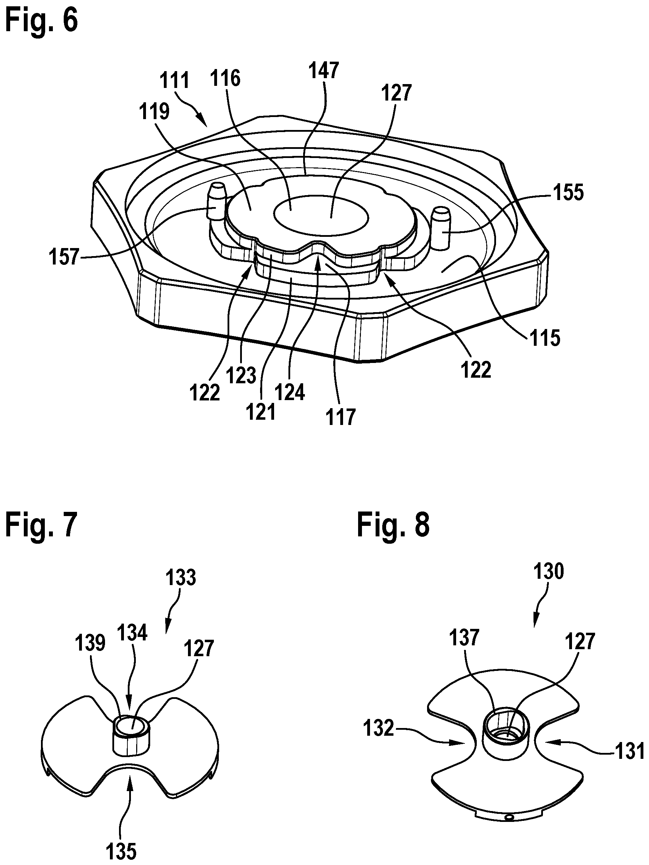

Das distalseitige Drehscheibenlager 130 weist eine erste Aussparung 131 und eine zweite Aussparung 132 auf, welche gegenüber angeordnet sind, sodass das distale Drehscheibenlager 130 eine Form mit zwei Flügeln aufweist. In der Mitte des distalen Drehscheibenlagers 130 ist ein optischer Durchgang 127 und um den optischen Durchgang 127 ein erstes Verbindungselement 137 angeordnet (Figur 8). Das proximale Drehscheibenlager 133 ist in seiner Grundform ähnlich mit einer dritten Aussparung 134 und einer gegenüberliegenden vierten Aussparung 135 ausgebildet (Figur 7). In seiner Mitte weist das proximale Drehscheibenlager 133 ebenfalls einen optischen Durchgang 127 und ein um den optischen Durchgang 127 angeordnetes zweites Verbindungselement 139 auf. Das erste Verbindungselement 137 des distalen Drehscheibenlagers 130 und das zweite Verbindungselement 139 des proximalen Drehscheibenlagers 133 sind komplementär zueinander ausgebildet, wobei im montierten Zustand das zweite Verbindungselement 139 in dem ersten Verbindungselement 137 aufgenommen ist. Dadurch ist eine formschlüssige Verbindung zwischen dem ersten Verbindungselement 137 und dem zweiten Verbindungselement 139 ausgebildet. Eine Außenoberfläche des ersten Verbindungselementes 137 ist im montierten Zustand von einer Lageraussparung 116 des Filterrades 111 umgeben.

In einer in Figur 9 gezeigten Alternative eines proximalen Drehscheibenlagers 133 sind die dritte Aussparung 134 und die vierte Aussparung 135 nicht wie in Figur 7 gezeigt am äußeren Rand des proximalen Drehscheibenlagers 133 angeordnet, sondern sind weiter innenliegend rundumlaufend und eine Plattenfläche der Alternative des proximalen Drehscheibenlagers 133 durchbrechend ausgebildet. Ein distales Drehscheibenlagers 130 ist in dieser Alternative äquivalent ausgebildet.

Die erste Aussparung 131 und die zweite Aussparung 132 des distalen Drehscheibenlagers 130 sowie die dritte Aussparung 134 und die vierte Aussparung 135 des proximalen Drehscheibenlagers 133 ermöglichen jeweils das Durchtreten eines langen Führungsbolzens 155 und/oder eines kurzen Führungsbolzens 157 der Filterhalter 141 bis 144 zur jeweiligen distalen Radfläche 113 oder proximalen Radfläche 115 des Filterrades 111.

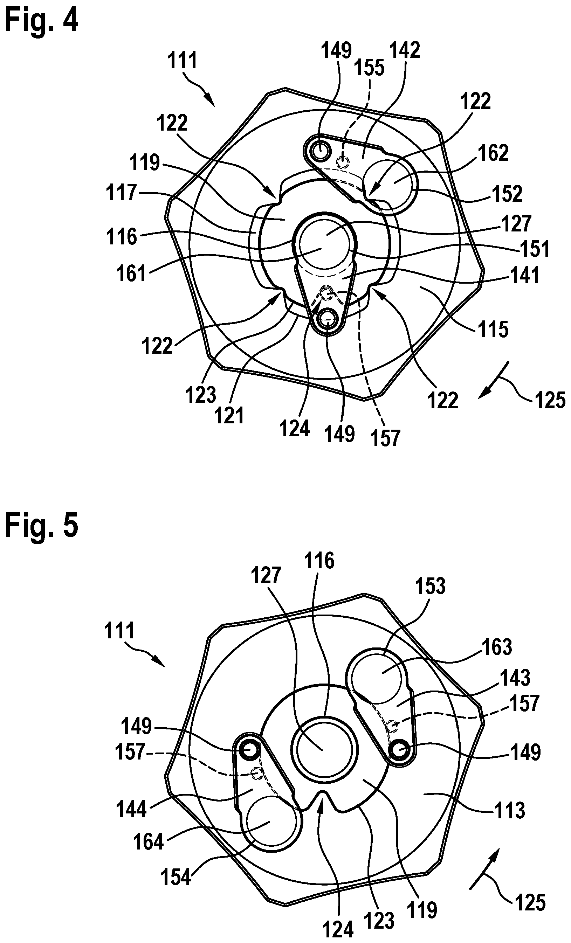

Das Filterrad 111 weist auf seiner proximalen Radfläche 115 innenliegend die Lageraussparung 116 mit einem größeren Durchmesser als der optische Durchgang 127 des Filterwechslers 101 auf. Um die Lageraussparung 116 ist direkt auf der proximalen Radfläche 115 eine erste Führungsebene 117 angeordnet. Die erste Führungsebene 117 weist an ihrem Umfang eine erste Kontaktfläche 121 auf, in welche vier gleichmäßig über den Umfang der ersten Führungsebene 117 verteilte erste Einschnitte 122 eingebracht sind. Auf der ersten Führungsebene 117 ist eine zweite Führungsebene 119 mit einem tiefen zweiten Einschnitt 124 in ihrer zweiten Kontaktfläche 123 angeordnet. Gegenüber diesen zweiten Einschnittes 124 ist eine Übergangsposition 147 angeordnet, an der beide Kontaktflächen frei von einem Einschnitt sind. Beim Anliegen des kurzen Führungsbolzen 157 an dieser Übergangsposition 147, sind alle Filterhalter 141, 142, 143, 144 aus dem optischen Durchgang 127 ausgeschwenkt. Des Weiteren sind in der zweiten Kontaktfläche 123 in der Figur 6 nicht näher gekennzeichnete kleinere Aussparungen korrespondierend zu den ersten Einschnitten 122 der ersten Führungsebene 117 angeordnet. Ein kurzer Führungsbolzen 157 liegt mit seiner Außenoberfläche seitlich an der zweiten Kontaktfläche 123 der zweiten Führungsebene 119 an. Ein langer Führungsbolzen 155 liegt an der ersten Kontaktfläche 121 der ersten Führungsebene 121 an. Der kurze Führungsbolzen 157 ist mit dem ersten Filterhalter 141 verbunden, in dessen erste Filteraufnahme 141 ein erstes Filter 161 für einen Fluoreszenzmodus aufgenommen ist. Der lange Führungsbolzen 155 ist mit dem langen zweiten Filterhalter 142 verbunden, in dessen zweiter Filteraufnahme 152 ein zweiter Filter 162 für einen Weißlichtmodus aufgenommen ist (Figur 4).

Auf seiner distalen Radfläche 113 weist das Filterrad 111 um die Lageraussparung 116 mit dem innenliegenden optischen Durchgang 127 eine zweite Führungsebene 119 als einzige Führungsebene auf, welche direkt auf der distalen Radfläche 113 angeordnet ist. Die zweite Führungsebene 119 der distalen Radfläche 113 ist ähnlich zur zweiten Führungsebene der proximalen Radfläche 115 ausgebildet, weist jedoch nur einen zweiten Einschnitt 124 in seiner zweiten Kontaktfläche 123 auf. Bis auf diesen zweiten Einschnitt 124 ist die Kontaktfläche 123 umlaufend kreisrund ausgebildet. An dieser Kontaktfläche 123 sind jeweils die kurzen Führungsbolzen 157 des dritten Filterhalters 143 und des vierten Filterhalters 144 angeordnet. Der dritte Filterhalter 143 weist in seiner dritten Filteraufnahme 153 ein drittes Filter 163 für einen anderen Fluoreszenzmodus und der vierte Filterhalter 144 weist in seiner vierten Filteraufnahme 154 ein viertes Filter 164 für einen weiteren Fluoreszenzmodus auf.

Mit dem Filterwechsler 101 und dem Endoskopsystem 171 werden folgende Arbeitsgänge durchgeführt.

Beim Drehen des Filterrades 111 durch einen Anwender mittels Kontaktieren der Außenoberfläche des Filterrades 111 außerhalb des Gehäuses 103 in einer Rotationsrichtung 125 im Uhrzeigersinn wird der lange Führungsbolzen 155 des langen zweiten Filterhalters 142 entlang der ersten Kontaktfläche 121 und der kurze Führungsbolzen 157 des ersten Filterhalters 141 entlang der zweiten Kontaktfläche 123 auf der Seite der proximalen Radfläche 115 bewegt (Figur 4). Auf der Seite der distalen Radfläche 113 bewegen sich entsprechend die beiden kurzen Führungsbolzen 157 des dritten Filterhalters 143 und des vierten Filterhalters 144 beide entlang der zweiten Kontaktfläche 123 der zweiten Führungsebene 119 (Figur 5). Der jeweilige kurze Führungsbolzen 157 und der lange Führungsbolzen 155 werden jeweils mittels der dem jeweiligen Filterhalter 141 bis 144 zugeordneten Torsionsfedern 165 gegen die jeweilige Kontaktfläche 121, 123 gedrückt, indem der jeweilige freie Schenkel der jeweiligen Torsionsfeder 165 in der jeweiligen Nut 164 gegen den zugehörigen Filterhalter 141 bis 144 drückt.

In dem in Figur 4 gezeigten Zustand, liegt der kurze Bolzen 157 des ersten Filterhalters 141 in dem zweiten Einschnitt 124 der zweiten Führungsebene 119 auf der proximalen Radfläche 115, sodass die erste Filteraufnahme 151 mit dem ersten Filter 161 in den optischen Durchgang 127 eingeschwenkt ist und dementsprechend ein Fluoreszenzmodus von dem Anwender verwendet wird. Währenddessen ist der zweite Filterhalter 142 ausgeschwenkt. Ebenso sind der dritte Filterhalter 143 und der vierte Filterhalter 144 an der gegenüberliegenden distalen Radfläche 113 ausgeschwenkt und befinden sich in einer Ausgangsposition.

Ausgehend von den in den Figuren 4 und 5 gezeigten Positionen der vier Filterhalter 141, 142, 143, 144 wird bei einem Drehen des Filterrades 111 in der Rotationsrichtung 125 an der proximalen Radfläche 115 der lange Führungsbolzen 155 entlang der ersten Kontaktfläche 121 in Rotationsrichtung 125 bis zum nachfolgenden nächsten ersten Einschnitt 122 gedreht, wobei zeitgleich sich der kurze Bolzen 157 entlang der zweiten Kontaktfläche 123 in der Rotationsrichtung 125 bewegt und durch Verlassen des zweiten Einschnittes 124 ausgeschwenkt wird. Der dritte Filterhalter 143 und der vierte Filterhalter 144 an der distalen Radfläche 113 werden bei Drehen in der Rotationsrichtung 125, welche aufgrund der entgegengesetzten Anordnung gegen den Uhrzeigersinn liegt, weitergedreht, bleiben aber am Außenumfang der zweiten Führungsebene 119 frei von dem zweiten Einschnitt 124. Somit schwenkt bei Erreichen des ersten Einschnitts 122 in Rotationsrichtung 125 im Uhrzeigersinn der lange Führungsbolzen 155 in den ersten Einschnitt 122 ein und folglich wird die zweite Filteraufnahme 152 mit dem zweiten Filter 162 für einen Weißlichtmodus in den optischen Durchgang 127 eingeschwenkt. Bei weiterem Drehen in der Rotationsrichtung 125 wird nachfolgend die vierte Filteraufnahme 154 des vierten Filterhalters 144 an der distalen Plattenfläche 133 in den optischen Durchgang 127 eingeschwenkt, wenn der kurze Führungsbolzen 157 den zweiten Einschnitt 124 erreicht. Beim weiteren Drehen in der Rotationsrichtung 125 wird aufgrund der vier gleichmäßig beabstandeten ersten Einschnitte 122 in der ersten Kontaktfläche 121 der ersten Führungsebene 117 auf der proximalen Radfläche 115 wieder der zweite lange Filterhalter 142 mit dem zweiten Filter 162 für den Weißlichtmodus eingeschnappt. Somit wird bei Weiterdrehen in der Rotationsrichtung 125 jeweils nach dem Einschwenken des ersten Filters 161, des dritten Filters 163 oder des vierten Filters 164 jeweils für einen Fluoreszenzmodus stets das der zweite Filterhalter 142 mit einem zweiten Filter 162 für einen Weißlichtmodus eingeschwenkt. Dadurch wird dem Anwender deutlich ein Wechsel zwischen den verschiedenen Fluoreszenzmodi und dem Weißlichtmodus angezeigt.

Somit wird ein Filterwechsler 101 bereitgestellt, mit welchem schnell, effizient und präzise nacheinander zwischen verschiedenen Filtern 161, 162, 163, 164 gewechselt werden kann und welcher mit verschiedenen Arten von Endoskopen 173 und Kameraköpfen 177 einsetzbar ist.

### Bezugszeichenliste

101 Filterwechsler
103 Gehäuse
105 Schraube
107 distale Deckelhälfte
109 proximale Deckelhälfte
111 Filterrad
113 distale Radfläche
115 proximale Radfläche
116 Lageraussparung
117 erste Führungsebene
119 zweite Führungsebene
121 erste Kontaktfläche
122 erster Einschnitt
123 zweite Kontaktfläche
124 zweiter Einschnitt
125 Rotationsrichtung
127 optischer Durchgang
129 optische Achse
130 distales Drehscheibenlager
131 erste Aussparung
132 zweite Aussparung
133 proximales Drehscheibenlager
134 dritte Aussparung
135 vierte Aussparung
137 erstes Verbindungselement
139 zweites Verbindungselement
141 erster Filterhalter
142 zweiter Filterhalter (lang)
143 dritter Filterhalter
144 vierter Filterhalter
145 Bohrung
146 Nut
147 Übergangsposition
149 Rotationsachse
151 erste Filteraufnahme
152 zweite Filteraufnahme
153 dritte Filteraufnahme
154 vierte Filteraufnahme
155 langer Führungsbolzen
157 kurzer Führungsbolzen
161 erster Filter (Fluoreszenz)
162 zweiter Filter (Weißlicht)
163 dritter Filter (Fluoreszenz)
164 vierter Filter (Fluoreszenz)
165 Torsionsfeder
167 Haltepin
171 Endoskopsystem
173 Endoskop
175 Endoskopaufnahme
177 Kamerakopf
179 Kameraaufnahme

## Patentansprüche

1. Filterwechselvorrichtung (101) für eine endoskopische Kamera, wobei die Filterwechselvorrichtung (101) mindestens ein erstes Gehäuseteil (107, 109), eine Drehplatte (111) mit zwei sich gegenüberliegenden Plattenflächen (113, 115), mindestens ein erstes Drehplattenlager (130, 133) und mindestens zwei schwenkbare Filterhalter (141, 142, 143, 144) mit jeweils einer Filteraufnahme (151, 152, 153, 154) für einen optischen Filter (161, 162, 163, 163) aufweist, wobei das erste Drehplattenlager (130, 133) zwischen dem ersten Gehäuseteil (107, 109) und einer ersten Plattenfläche (113, 115) der Drehplatte (111) angeordnet ist, die Drehplatte (111) relativ zum mindestens ersten Gehäuseteil (107, 109) drehbar ist und das mindestens erste Gehäuseteil (107, 109), das mindestens erste Drehplattenlager (130, 133) und die Drehplatte (111) jeweils einen optischen Durchgang (127) entlang einer optischen Achse (129) aufweisen, wobei die mindestens zwei schwenkbaren Filterhalter (141, 142, 143, 144) jeweils mittels einer Rotationsachse (149) drehbar an dem ersten Gehäuseteil (107, 109) angeordnet sind und jeweils ein Führungselement (155, 157) ausgerichtet zur Drehplatte (111) aufweisen, **dadurch gekennzeichnet, dass** das mindestens erste Drehplattenlager (130, 133) zwei Aussparungen (131, 132, 134, 135) zum Durchführen von jeweils einem Führungselement (155, 157) aufweist und die Drehplatte (111) auf ihrer ersten Plattenfläche (113, 115) mindestens eine erste Führungsebene (117, 119) mit einer geformten, umlaufenden Kontaktfläche (121, 123) an einem Umfang aufweist, sodass beim Drehen der Drehplatte (111) durch eine Druckkraft der geformten, umlaufenden Kontaktfläche (121) auf zumindest einem der beiden Führungselemente (155, 157) der zugehörige Filterhalter (141, 142, 143, 144) in oder aus den optischen Durchgang (127) schwenkbar oder in einer Ausgangsposition frei von dem optischen Durchgang (127) positionierbar ist.

2. Filterwechselvorrichtung (101) nach Anspruch 1, **dadurch gekennzeichnet, dass** die Filterwechselvorrichtung (101) zugeordnet zu jedem Filterhalter (141, 142, 143, 144) mindestens jeweils ein federndes Element (165) aufweist, wobei mittels des mindestens einen federnden Elementes (165) derart auf den jeweiligen Filterhalter (141, 142, 143, 144) einwirkbar ist, dass eine Federkraft des mindestens einen federnden Elementes (165) entgegen der Druckkraft der geformten, umlaufenden Kontaktfläche (121, 123) gerichtet ist.

3. Filterwechselvorrichtung (101) nach Anspruch 1 oder 2, **dadurch gekennzeichnet, dass** die Filterwechselvorrichtung (101) mindestens ein zweites Gehäuseteil (107, 109), mindestens ein zweites Drehplattenlager (130, 133) und mindestens einen weiteren schwenkbaren Filterhalter (141, 142, 143, 144), insbesondere zwei weitere schwenkbare Filterhalter, angeordnet an dem zweiten Gehäuseteil (107, 109) aufweist, wobei das zweite Drehplattenlager (130, 133) zwischen dem zweiten Gehäuseteil (107, 109) und einer zweiten Plattenfläche (113, 115) der Drehplatte (111) angeordnet ist und die Drehplatte (111) auf ihrer zweiten Plattenfläche (113, 115) mindestens eine erste Führungsebene (117, 119) mit einer geformten, umlaufenden Kontaktfläche (121, 123) an einem Umfang aufweist.

4. Filterwechselvorrichtung (101) nach einem der vorherigen Ansprüche, **dadurch gekennzeichnet, dass** die erste Plattenfläche und/oder die zweite Plattenfläche (113, 115) eine zweite Führungsebene (117, 119) mit einer geformten, umlaufenden Kontaktfläche aufweist oder aufweisen, sodass ein erstes Führungselement (155) entlang der ersten Kontaktfläche (121) der ersten Führungsebene (117) und ein zweites Führungselement (157) entlang der zweiten Kontaktfläche (123) der zweiten Führungsebenes (119) jeweils der ersten Plattenfläche (113) und/oder der zweiten Plattenfläche (115) bewegbar sind.

5. Filterwechselvorrichtung (101) nach Anspruch 4, **dadurch gekennzeichnet, dass** die erste Führungsebene (117) und die zweite Führungsebene (119) der ersten Plattenfläche (113) und/oder der zweiten Plattenfläche (115) unterschiedliche Durchmesser und/oder unterschiedlich geformte, umlaufende Kontaktflächen (121, 123) aufweisen.

6. Filterwechselvorrichtung (101) einem der vorherigen Ansprüche, **dadurch gekennzeichnet, dass** die Filterhalter (141, 142, 143, 144) eine gleiche Länge und/oder eine unterschiedliche Länge aufweisen.

7. Filterwechselvorrichtung (101) nach einem der vorherigen Ansprüche, **dadurch gekennzeichnet, dass** die erste Führungsebene (117) und/oder die zweite Führungsebene (119) jeweils mindestens eine Aussparung (122, 124) in ihrer Kontaktfläche (121, 123) aufweisen, sodass bei Anordnung eines Führungselementes (155, 157) in der Aussparung (122, 124) die Filteraufnahme (151, 152) des zugehörigen Filterhalters (141, 142) in den optischen Durchgang (127) der Drehplatte (111) präzise einschwenkbar ist.

8. Filterwechselvorrichtung (101) nach einem der vorherigen Ansprüche, **dadurch gekennzeichnet, dass** die erste Kontaktfläche (121) der ersten Führungsebene (117), die zweite Kontaktfläche (123) der zweiten Führungsebene (119), die mindestens eine Aussparung oder die Aussparungen (122, 124) symmetrisch ausgebildet und/oder angeordnet ist oder sind.

9. Filterwechselvorrichtung (101) nach einem der Ansprüche 3 bis 8, **dadurch gekennzeichnet, dass** die erste Plattenfläche (113) und die zweite Plattenfläche (115) jeweils eine zweite Führungsebene (119) mit jeweils einer Aussparung (124) zum Einschwenken eines Filterhalters (141, 143, 144) mit einem ersten optischen Filter (161, 163, 164) aufweist, und eine der beiden Plattenflächen (113, 115) eine erste Führungsebene (117) mit vier gleichmäßig beabstandeten Aussparungen (122) zum Einschwenken eines Filterhalters (142) mit einem zweiten optischen Filter (162) aufweist, sodass jeweils eines der erstes optischen Filter (161, 163, 164) und das zweite optische Filter (162) nacheinander abwechselnd bei einer Umdrehung der Drehplatte (111) einschwenkbar sind.

10. Filterwechselvorrichtung (101) nach einem der vorherigen Ansprüche, **dadurch gekennzeichnet, dass** die erste Führungsebene (117) und/oder die zweite Führungsebene (119) der ersten Plattenfläche (113) und/oder der zweiten Plattenfläche (115) derart ausgebildet sind, dass in einer Übergangsposition (147) der optische Durchgang (127) frei von einem eingeschwenkten Filterhalter (141, 142, 143, 144) ist.

11. Filterwechselvorrichtung (101) nach einem der vorherigen Ansprüche, **dadurch gekennzeichnet, dass** das erste Drehplattenlager (130) ein erstes Verbindungselement (137) und das zweite Drehplattenlager (133) ein zweites Verbindungselement (139) aufweist, wobei der erste Verbindungselement (137) und das zweite Verbindungselement (139) formschlüssig und/oder kraftschlüssig miteinander verbindbar sind.

12. Filterwechselvorrichtung (101) nach einem der Ansprüche 3 bis 11, **dadurch gekennzeichnet, dass** das erste Gehäuseteil (107) und das zweite Gehäuseteil (109) in ihrem Inneren miteinander verbunden sind, sodass die Drehplatte (111) über ihrem gesamten Umfang von außen frei kontaktierbar und/oder drehbar ist.

13. Filterwechselvorrichtung (101) nach einem der Ansprüche 3 bis 11, **dadurch gekennzeichnet, dass** das erste Gehäuseteil (107) und das zweite Gehäuseteil (109) ein hermetisch dichtes Gehäuse (103) ausbilden, wobei die Drehplatte (111) innerhalb des Gehäuses (103) angeordnet ist und mindestens ein Gegenmagnetelement aufweist, sodass ein Drehen der Drehplatte (111) mittels einer außerhalb des Gehäuses zugeordneten Magneteinrichtung bewirkbar ist.

14. Kamerakopf (177) für ein Endoskop (173), wobei der Kamerakopf (177) einen Bildsensor, eine Öffnung zum Empfangen von Licht eines Bildes entlang eines optischen Pfades und ein optisches Linsensystem zum Fokussieren des Lichtes auf den Bildsensor aufweist, **dadurch gekennzeichnet, dass** der Kamerakopf (177) zumindest eine Filterwechselvorrichtung (101) nach einem der Ansprüche 1 bis 13 aufweist.

15. Nachrüstsatz zum Nachrüsten eines Kamerakopfes und/oder eines Endoskopes, **dadurch gekennzeichnet, dass** der Nachrüstsatz zumindest eine Filterwechselvorrichtung (101) nach einem der Ansprüche 1 bis 13 aufweist, sodass die Filterwechselvorrichtung (101) zwischen einem proximalen Ende des Endoskopes (173) und einem distalen Ende des Kamerakopfes (177) anordenbar ist.
